# EUROPÄISCHE PATENTSCHRIFT

(11) **EP 1 711 600 B1**
(45) Veröffentlichungstag und Bekanntmachung des Hinweises auf die Patenterteilung: **03.06.2009**
(21) Anmeldenummer: 05726334.5
(22) Anmeldetag: 04.02.2005
(51) Int. Cl.: C12N 9/12

(54) **MUTIERTE DNA-POLYMERASEN MIT ERHÖHTER FEHLPAARUNGS-DISKRIMINIRUNG**
MUTATED DNA POLYMERASES WITH INCREASED MISPAIRING DISCRIMINATION
ADN POLYMERASES MUTANTES PRESENTANT UNE DISCRIMINATION DE MESAPPARIEMENTS AUGMENTEE

(30) Priorität: 05.02.2004 DE 102004005885
(43) Veröffentlichungstag der Anmeldung: 18.10.2006
(73) Patentinhaber: Rheinische Friedrich-Wilhelms-Universität Bonn, 53113 Bonn (DE)
(72) Erfinder: MARX, Andreas, 78462 Konstanz (DE); SUMMERER, Daniel, 50823 Mannheim (DE); RUDINGER, Nicolaus, Zackes, 50823 Köln (DE)
(74) Vertreter: Helbing, Jörg
(86) Internationale Anmeldenummer: PCT/EP2005/050479
(87) Internationale Veröffentlichungsnummer: WO 2005/074350

(56) Entgegenhaltungen:
- MINNICK T ET AL.: "side chains that influence fidelity at the polymerase active site of Escherichia coli DNA polymerase I (klenow fragment)" THE JOURNAL OF BIOLOGICAL CHEMISTRY, Bd. 275, Nr. 5, 29. Januar 1999 (1999-01-29), Seiten 3067-3075, XP002341285
- PAVLOV A. ET AL: "Recent developments in the optimization of thermostable DNA polymerases for efficient applications" TRENDS IN BIOTECHNOLOGY, Bd. 22, Nr. 5, Mai 2004 (2004-05), Seiten 253-259, XP004504365
- PATEL P. ET AL.: "Prokaryotic DNA polymerase I: evolution, structure, and "base flipping" mechanism for nucleotide selection" J. MOL. BIOL., Bd. 308, 2001, Seiten 823-837, XP004466165

### SEQUENZPROTOKOLL

<110> Rheinische Friedrich-Wilhelms Universität Bonn
<120> Mutierte DNA-Polymerasen mit erhöhter Fehlpaarungs-Diskriminierung
<130> 050188wo JH/PCH
<140>
   <141>
<150> DE 102004005885.7
   <151> 2004-02-05
<160> 29
<170> PatentIn Ver. 2.1
<210> 1
   <211> 2787
   <212> DNA
   <213> Künstliche Sequenz
<220>
   <223> Beschreibung der künstlichen Sequenz: E. coli Wildtyp Klenow Fragment der DNA Polymerase 1
<400> 1
   atggttcaga tcccccaaaa tccacttatc cttgtagatg gttcatctta tctttatcgc 60
   gcatatcacg cgtttccccc gctgactaac agcgcaggcg agccgaccgg tgcgatgtat 120
   ggtgtcctca acatgctgcg cagtctgatc atgcaatata aaccgacgca tgcagcggtg 180
   gtctttgacg ccaagggaaa aacctttcgt gatgaactgt ttgaacatta caaatcacat 240
   cgcccgccaa tgccggacga tctgcgtgca caaatcgaac ccttgcacgc gatggttaaa 300
   gcgatgggac tgccgctgct ggcggtttct ggcgtagaag cggacgacgt tatcggtact 360
   ctggcgcgcg aagccgaaaa agccgggcgt ccggtgctga tcagcactgg cgataaagat 420
   atggcgcagc tggtgacgcc aaatattacg cttatcaata ccatgacgaa taccatcctc 480
   ggaccggaag aggtggtgaa taagtacggc gtgccgccag aactgatcat cgatttcctg 540
   gcgctgatgg gtgactcctc tgataacatt cctggcgtac cgggcgtcgg tgaaaaaacc 600
   gcgcaggcat tgctgcaagg tcttggcgga ctggatacgc tgtatgccga gccagaaaaa 660
   attgctgggt tgagcttccg tggcgcgaaa acaatggcag cgaagctcga gcaaaacaaa 720
   gaagttgctt atctctcata ccagctggcg acgattaaaa ccgacgttga actggagctg 780
   acctgtgaac aactggaagt gcagcaaccg gcagcggaag agttgttggg gctgttcaaa 840
   aagtatgagt tcaaacgctg gactgctgat gtcgaagcgg gcaaatggtt acaggccaaa 900
   ggggcaaaac cagccgcgaa gccacaggaa accagtgttg cagacgaagc accagaagtg 960
   acggcaacgg tgatttctta tgacaactac gtcaccatcc ttgatgaaga aacactgaaa 1020
   gcgtggattg cgaagctgga aaaagcgccg gtatttgcat ttgataccga aaccgacagc 1080
   cttgataaca tctctgctaa cctggtcggg ctttcttttg ctatcgagcc aggcgtagcg 1140
   gcatatattc cggttgctca tgattatctt gatgcgcccg atcaaatctc tcgcgagcgt 1200
   gcactcgagt tgctaaaacc gctgctggaa gatgaaaagg cgctgaaggt cgggcaaaac 1260
   ctgaaatacg atcgcggtat tctggcgaac tacggcattg aactgcgtgg gattgcgttt 1320
   gataccatgc tggagtccta cattctcaat agcgttgccg ggcgtcacga tatggacagc 1380
   ctcgcggaac gttggttgaa gcacaaaacc atcacttttg aagagattgc tggtaaaggc 1440
   aaaaatcaac tgacctttaa ccagattgcc ctcgaagaag ccggacgtta cgccgccgaa 1500
   gatgcagatg tcaccttgca gttgcatctg aaaatgtggc cggatctgca aaaacacaaa 1560
   gggccgttga acgtcttcga gaatatcgaa atgccgctgg tgccggtgct ttcacgcatt 1620
   gaacgtaacg gtgtgaagat cgatccgaaa gtgctgcaca atcattctga agagctcacc 1680
   cttcgtctgg ctgagctgga aaagaaagcg catgaaattg caggtgagga atttaacctt 1740
   tcttccacca agcagttaca aaccattctc tttgaaaaac agggcattaa accgctgaag 1800
   aaaacgccgg gtggcgcgcc gtcaacgtcg gaagaggtac tggaagaact ggcgctggac 1860
   tatccgttgc caaaagtgat tctggagtat cgtggtctgg cgaagctgaa atcgacctac 1920
   accgacaagc tgccgctgat gatcaacccg aaaaccgggc gtgtgcatac ctcttatcac 1980
   caggcagtaa ctgcaacggg acgtttatcg tcaaccgatc ctaacctgca aaacattccg 2040
   gtgcgtaacg aagaaggtcg tcgtatccgc caggcgttta ttgcgccaga ggattatgtg 2100
   attgtctcag cggactactc gcagattgaa ctgcgcatta tggcgcatct ttcgcgtgac 2160
   aaaggcttgc tgaccgcatt cgcggaagga aaagatatcc accgggcaac ggcggcagaa 2220
   gtgtttggtt tgccactgga aaccgtcacc agcgagcaac gccgtagcgc gaaagcgatc 2280
   aactttggtc tgatttatgg catgagtgct ttcggtctgg cgcggcaatt gaacattcca 2340
   cgtaaagaag cgcagaagta catggacctt tacttcgaac gctaccctgg cgtgctggag 2400
   tatatggaac gcacccgtgc tcaggcgaaa gagcagggct acgttgaaac gctggacgga 2460
   cgccgtctgt atctgccgga tatcaaatcc agcaatggtg ctcgtcgtgc agcggctgaa 2520
   cgtgcagcca ttaacgcgcc aatgcaggga accgccgccg acattatcaa acgggcgatg 2580
   attgccgttg atgcgtggtt acaggctgag caaccgcgtg tacgtatgat catgcaggta 2640
   cacgatgaac tggtatttga agttcataaa gatgatgttg atgccgtcgc gaagcagatt 2700
   catcaactga tggaaaactg tacccgtctg gatgtgccgt tgctggtgga agtggggagt 2760
   ggcgaaaact gggatcaggc gcactaa                                     2787
<210> 2
   <211> 928
   <212> PRT
   <213> Künstliche Sequenz
<220>
   <223> Beschreibung der künstlichen Sequenz: E.coli Klenow Fragment der DNA Polymerase 1
<400> 2
<210> 3
   <211> 2499
   <212> DNA
   <213> Künstliche Sequenz
<220>
   <223> Beschreibung der künstlichen Sequenz:Wildtyp Taq Polymerase
<400> 3
   atgaggggga tgctgcccct ctttgagccc aagggccggg tcctcctggt ggacggccac 60
   cacctggcct accgcacctt ccacgccctg aagggcctca ccaccagccg gggggagccg 120
   gtgcaggcgg tctacggctt cgccaagagc ctcctcaagg ccctcaagga ggacggggac 180
   gcggtgatcg tggtctttga cgccaaggcc ccctccttcc gccacgaggc ctacgggggg 240
   tacaaggcgg gccgggcccc cacgccggag gactttcccc ggcaactcgc cctcatcaag 300
   gagctggtgg acctcctggg gctggcgcgc ctcgaggtcc cgggctacga ggcggacgac 360
   gtcctggcca gcctggccaa gaaggcggaa aaggagggct acgaggtccg catcctcacc 420
   gccgacaaag acctttacca gctcctttcc gaccgcatcc acgtcctcca ccccgagggg 480
   tacctcatca ccccggcctg gctttgggaa aagtacggcc tgaggcccga ccagtgggcc 540
   gactaccggg ccctgaccgg ggacgagtcc gacaaccttc ccggggtcaa gggcatcggg 600
   gagaagacgg cgaggaagct tctggaggag tgggggagcc tggaagccct cctcaagaac 660
   ctggaccggc tgaagcccgc catccgggag aagatcctgg cccacatgga cgatctgaag 720
   ctctcctggg acctggccaa ggtgcgcacc gacctgcccc tggaggtgga cttcgccaaa 780
   aggcgggagc ccgaccggga gaggcttagg gcctttctgg agaggcttga gtttggcagc 840
   ctcctccacg agttcggcct tctggaaagc cccaaggccc tggaggaggc cccctggccc 900
   ccgccggaag gggccttcgt gggctttgtg ctttcccgca aggagcccat gtgggccgat 960
   cttctggccc tggccgccgc cagggggggc cgggtccacc gggcccccga gccttataaa 1020
   gccctcaggg acctgaagga ggcgcggggg cttctcgcca aagacctgag cgttctggcc 1080
   ctgagggaag gccttggcct cccgcccggc gacgacccca tgctcctcgc ctacctcctg 1140
   gacccttcca acaccacccc cgagggggtg gcccggcgct acggcgggga gtggacggag 1200
   gaggcggggg agcgggccgc cctttccgag aggctcttcg ccaacctgtg ggggaggctt 1260
   gagggggagg agaggctcct ttggctttac cgggaggtgg agaggcccct ttccgctgtc 1320
   ctggcccaca tggaggccac gggggtgcgc ctggacgtgg cctatctcag ggccttgtcc 1380
   ctggaggtgg ccgaggagat cgcccgcctc gaggccgagg tcttccgcct ggccggccac 1440
   cccttcaacc tcaactcccg ggaccagctg gaaagggtcc tctttgacga gctagggctt 1500
   cccgccatcg gcaagacgga gaagaccggc aagcgctcca ccagcgccgc cgtcctggag 1560
   gccctccgcg aggcccaccc catcgtggag aagatcctgc agtaccggga gctcaccaag 1620
   ctgaagagca cctacattga ccccttgccg gacctcatcc accccaggac gggccgcctc 1680
   cacacccgct tcaaccagac ggccacggcc acgggcaggc taagtagctc cgatcccaac 1740
   ctccagaaca tccccgtccg caccccgctt gggcagagga tccgccgggc cttcatcgcc 1800
   gaggaggggt ggctattggt ggccctggac tatagccaga tagagctcag ggtgctggcc 1860
   cacctctccg gcgacgagaa cctgatccgg gtcttccagg aggggcggga catccacacg 1920
   gagaccgcca gctggatgtt cggcgtcccc cgggaggccg tggaccccct gatgcgccgg 1980
   gcggccaaga ccatcaactt cggggtcctc tacggcatgt cggcccaccg cctctcccag 2040
   gagctagcca tcccttacga ggaggcccag gccttcattg agcgctactt tcagagcttc 2100
   cccaaggtgc gggcctggat tgagaagacc ctggaggagg gcaggaggcg ggggtacgtg 2160
   gagaccctct tcggccgccg ccgctacgtg ccagacctag aggcccgggt gaagagcgtg 2220
   cgggaggcgg ccgagcgcat ggccttcaac atgcccgtcc agggcaccgc cgccgacctc 2280
   atgaagctgg ctatggtgaa gctcttcccc aggctggagg aaatgggggc caggatgctc 2340
   cttcaggtcc acgacgagct ggtcctcgag gccccaaaag agagggcgga ggccgtggcc 2400
   cggctggcca aggaggtcat ggagggggtg tatcccctgg ccgtgcccct ggaggtggag 2460
   gtggggatag gggaggactg gctctccgcc aaggagtga                       2499
<210> 4
   <211> 832
   <212> PRT
   <213> Künstliche Sequenz
<220>
   <223> Beschreibung der künstlichen Sequenz: Wildtyp Taq Polymerase
<400> 4
<210> 5
   <211> 39
   <212> DNA
   <213> Künstliche Sequenz
<220>
   <223> Beschreibung der künstlichen Sequenz: Primer
<400> 5
   gtacgtatga tcatgnnnnn nnnngatgaa ctggtattt 39
<210> 6
   <211> 23
   <212> DNA
   <213> Künstliche Sequenz
<220>
   <223> Beschreibung der künstlichen Sequenz: Downstream-Primer
<400> 6
   gctaattaag cttggctgca ggc 23
<210> 7
   <211> 24
   <212> DNA
   <213> Künstliche Sequenz
<220>
   <223> Beschreibung der künstlichen Sequenz: Antisense-Primer
<400> 7
   tacatggacc tttacttcga acgc 24
<210> 8
   <211> 20
   <212> DNA
   <213> Künstlich Sequenz
<220>
   <223> Beschreibung der künstlichen Sequenz: Primer FVL20TH
<400> 8
   acaaaatacc tgtattcctt 20
<210> 9
   <211> 90
   <212> DNA
   <213> Künstliche Sequenz
<220>
   <223> Beschreibung der künstlichen Sequenz: Matrize TFVL90A
<400> 9
   gacatcatga gagacatcgc ctctgggcta ataggactac ttctaatctg taagagcaga 60
   tccctggaca ggcaaggaat acaggtattt 90
<210> 10
   <211> 90
   <212> DNA
   <213> Künstliche Sequenz
<220>
   <223> Beschreibung der künstlichen Sequenz: Matrize TFVL90G
<400> 10
   gacatcatga gagacatcgc ctctgggcta ataggactac ttctaatctg taagagcaga 60
   tccctggaca ggcgaggaat acaggtattt 90
<210> 11
   <211> 20
   <212> DNA
   <213> Künstliche Sequenz
<220>
   <223> Beschreibung der künstlichen Sequenz: Primer zur Detektion des SNPs in der humanen genomischen Factor-V-Leiden-DNA-Sequenz
<400> 11
   acaaaatacc tgtattcctn 20
<210> 12
   <211> 35
   <212> DNA
   <213> Künstliche Sequenz
<220>
   <223> Beschreibung der künstlichen Sequenz: Matrize der humanen genomischen Factor-V-Leiden-DNA-Sequenz; n=g, Wildtypmatrize; n=a, mutante Matrize
<400> 12
   gatccctgga caggcnagga atacaggtat tttgt 35
<210> 13
   <211> 22
   <212> DNA
   <213> Künstliche Sequenz
<220>
   <223> Beschreibung der künstlichen Sequenz: Primer zur Detektion der humanen somatische BRAF-T1796A-Mutation
<400> 13
   gacccactcc atcgagattt ct 22
<210> 14
   <211> 35
   <212> DNA
   <213> Künstliche Sequenz
<220>
   <223> Beschreibung der künstlichen Sequenz: Wildtyp Matrize des BRAF Gens; w= t, Wildtypmatrize; w = a, mutante Matrize
<400> 14
   ggtctagcta cagwgaaatc tcgatggagt gggtc 35
<210> 15
   <211> 25
   <212> DNA
   <213> Künstliche Sequenz
<220>
   <223> Beschreibung der künstlichen Sequenz: Primer zur Detektion der humanen Dihydropyrimidin-Dehydrogenase (DPyD) Mutation G735A
<400> 15
   gttttagatg ttaaatcaca cttat 25
<210> 16
   <211> 38
   <212> DNA
   <213> Künstliche Sequenz
<220>
   <223> Beschreibung der künstlichen Sequenz: Matrize des humanen DPyD; r = g, Wildtypmatrize; r = a, mutante Matrize
<400> 16
   ctttccagac aacrtaagtg tgatttaaca tctaaaac 38
<210> 17
   <211> 20
   <212> DNA
   <213> Künstliche Sequenz
<220>
   <223> Beschreibung der künstlichen Sequenz: Primer zur Detektion der humanen sauren Ceramidase Mutation A107G
<400> 17
   cgttggtcct gaaggaggat 20
<210> 18
   <211> 33
   <212> DNA
   <213> Künstliche Sequenz
<220>
   <223> Beschreibung der künstlichen Sequenz: Matrize der humanen sauren Ceramidase; r = a, Wildtypmatrize; r = g, mutante Matrize
<400> 18
   aaatcaacct rtcctccttc aggaccaacg tac 33
<210> 19
   <211> 22
   <212> DNA
   <213> Künstliche Sequenz
<220>
   <223> Beschreibung der künstlichen Sequenz: Primersonde BrafT
<400> 19
   gacccactcc atcgagattt ct 22
<210> 20
   <211> 22
   <212> DNA
   <213> Künstliche Sequenz
<220>
   <223> Beschreibung der künstlichen Sequenz: umgekehrter Primer für BRAF
<400> 20
   agaggaaaga tgaagtacta tg 22
<210> 21
   <211> 239
   <212> DNA
   <213> Künstliche Sequenz
<220>
   <223> Beschreibung der künstlichen Sequenz: Zielmatrize BrafX; w = a, Braf A (Wildtyp); w = t, BrafT (Mutante)
<400> 21
   caactgttca aactgatggg acccactcca tcgagatttc wctgtagcta gaccaaaatc 60
   acctattttt actgtgaggt cttcatgaag aaatatatct gaggtgtagt aagtaaagga 120
   aaacagtaga tctcattttc ctatcagagc aagcattatg aagagtttag gtaagagatc 180
   taatttctat aattctgtaa tataatattc tttaaaacat agtacttcat ctttcctct 239
<210> 22
   <211> 25
   <212> DNA
   <213> Künstliche Sequenz
<220>
   <223> Beschreibung der künstlichen Sequenz: Primersonde DpyDT
<400> 22
   gttttagatg ttaaatcaca cttat 25
<210> 23
   <211> 23
   <212> DNA
   <213> Künstliche Sequenz
<220>
   <223> Beschreibung der künstlichen Sequenz: umgekehrter Primer für DpyDT
<400> 23
   aaagctcctt tctgaatatt gag 23
<210> 24
   <211> 300
   <212> DNA
   <213> Künstliche Sequenz
<220>
   <223> Beschreibung der künstlichen Sequenz: Zielmatrize DpyDX; r = a, DpyDA (Wildtyp); r = t, DpyDT (Mutante)
<400> 24
   aaaatgtgag aagggacctc ataaaatatg tcatatggaa atgagcagat aataaagatt 60
   atagcttttc tttgtcaaaa ggagactcaa tatctttact ctttcatcag gacattgtga 120
   caaatgtttc ccccagaatc atccggggaa ccacctctgg ccccatgtat ggccctggac 180
   aaagctcctt tctgaatatt gagctcatca gtgagaaaac ggctgcatat tggtgtcaaa 240
   gtgtcactga actaaaggct gactttccag acaacrtaag tgtgatttaa catctaaaac 300
<210> 25
   <211> 7043
   <212> DNA
   <213> Künstliche Sequenz
<220>
   <223> Beschreibung der künstlichen Sequenz: pTTQ18::Taq
<400> 25
   gaactggatc tcaacagcgg taagatcctt gagagttttc gccccgaaga acgttttcca 60
   atgatgagca cttttaaagt tctgctatgt ggcgcggtat tatcccgtat tgacgccggg 120
   caagagcaac tcggtcgccg catacactat tctcagaatg acttggttga gtactcacca 180
   gtcacagaaa agcatcttac ggatggcatg acagtaagag aattatgcag tgctgccata 240
   accatgagtg ataacactgc ggccaactta cttctgacaa cgatcggagg accgaaggag 300
   ctaaccgctt ttttgcacaa catgggggat catgtaactc gccttgatcg ttgggaaccg 360
   gagctgaatg aagccatacc aaacgacgag cgtgacacca cgatgcctgt agcaatggca 420
   acaacgttgc gcaaactatt aactggcgaa ctacttactc tagcttcccg gcaacaatta 480
   atagactgga tggaggcgga taaagttgca ggaccacttc tgcgctcggc ccttccggct 540
   ggctggttta ttgctgataa atctggagcc ggtgagcgtg ggtctcgcgg tatcattgca 600
   gcactggggc cagatggtaa gccctcccgt atcgtagtta tctacacgac ggggagtcag 660
   gcaactatgg atgaacgaaa tagacagatc gctgagatag gtgcctcact gattaagcat 720
   tggtaactgt cagaccaagt ttactcatat atactttaga ttgatttaaa acttcatttt 780
   taatttaaaa ggatctaggt gaagatcctt tttgataatc tcatgaccaa aatcccttaa 840
   cgtgagtttt cgttccactg agcgtcagac cccgtagaaa agatcaaagg atcttcttga 900
   gatccttttt ttctgcgcgt aatctgctgc ttgcaaacaa aaaaaccacc gctaccagcg 960
   gtggtttgtt tgccggatca agagctacca actctttttc cgaaggtaac tggcttcagc 1020
   agagcgcaga taccaaatac tgtccttcta gtgtagccgt agttaggcca ccacttcaag 1080
   aactctgtag caccgcctac atacctcgct ctgctaatcc tgttaccagt ggctgctgcc 1140
   agtggcgata agtcgtgtct taccgggttg gactcaagac gatagttacc ggataaggcg 1200
   cagcggtcgg gctgaacggg gggttcgtgc acacagccca gcttggagcg aacgacctac 1260
   accgaactga gatacctaca gcgtgagcat tgagaaagcg ccacgcttcc cgaagggaga 1320
   aaggcggaca ggtatccggt aagcggcagg gtcggaacag gagagcgcac gagggagctt 1380
   ccagggggaa acgcctggta tctttatagt cctgtcgggt ttcgccacct ctgacttgag 1440
   cgtcgatttt tgtgatgctc gtcagggggg cggagcctat ggaaaaacgc cagcaacgcg 1500
   gcctttttac ggttcctggc cttttgctgg ccttttgctc acatgttctt tcctgcgtta 1560
   tcccctgatt ctgtggataa ccgtattacc gcctttgagt gagctgatac cgctcgccgc 1620
   agccgaacga ccgagcgcag cgagtcagtg agcgaggaag cggaagagcg cccaatacgc 1680
   aaaccgcctc tccccgcgcg ttggccgatt cattaatgca gaattaattc tcatgtttga 1740
   cagcttatca tcgactgcac ggtgcaccaa tgcttctggc gtcaggcagc catcggaagc 1800
   tgtggtatgg ctgtgcaggt cgtaaatcac tgcataattc gtgtcgctca aggcgcactc 1860
   ccgttctgga taatgttttt tgcgccgaca tcataacggt tctggcaaat attctgaaat 1920
   gagctgttga caattaatca tcggctcgta taatgtgtgg aattgtgagc ggataacaat 1980
   ttcacacagg aaacagcgat gaattcgggg atgctgcccc tctttgagcc caagggccgg 2040
   gtcctcctgg tggacggcca ccacctggcc taccgcacct tccacgccct gaagggcctc 2100
   accaccagcc ggggggagcc ggtgcaggcg gtctacggct tcgccaagag cctcctcaag 2160
   gccctcaagg aggacgggga cgcggtgatc gtggtctttg acgccaaggc cccctccttc 2220
   cgccacgagg cctacggggg gtacaaggcg ggccgggccc ccacgccgga ggactttccc 2280
   cggcaactcg ccctcatcaa ggagctggtg gacctcctgg ggctggcgcg cctcgaggtc 2340
   ccgggctacg aggcggacga cgtcctggcc agcctggcca agaaggcgga aaaggagggc 2400
   tacgaggtcc gcatcctcac cgccgacaaa gacctttacc agctcctttc cgaccgcatc 2460
   cacgtcctcc accccgaggg gtacctcatc accccggcct ggctttggga aaagtacggc 2520
   ctgaggcccg accagtgggc cgactaccgg gccctgaccg gggacgagtc cgacaacctt 2580
   cccggggtca agggcatcgg ggagaagacg gcgaggaagc ttctggagga gtgggggagc 2640
   ctggaagccc tcctcaagaa cctggaccgg ctgaagcccg ccatccggga gaagatcctg 2700
   gcccacatgg acgatctgaa gctctcctgg gacctggcca aggtgcgcac cgacctgccc 2760
   ctggaggtgg acttcgccaa aaggcgggag cccgaccggg agaggcttag ggcctttctg 2820
   gagaggcttg agtttggcag cctcctccac gagttcggcc ttctggaaag ccccaaggcc 2880
   ctggaggagg ccccctggcc cccgccggaa ggggccttcg tgggctttgt gctttcccgc 2940
   aaggagccca tgtgggccga tcttctggcc ctggccgccg ccaggggggg ccgggtccac 3000
   cgggcccccg agccttataa agccctcagg gacctgaagg aggcgcgggg gcttctcgcc 3060
   aaagacctga gcgttctggc cctgagggaa ggccttggcc tcccgcccgg cgacgacccc 3120
   atgctcctcg cctacctcct ggacccttcc aacaccaccc ccgagggggt ggcccggcgc 3180
   tacggcgggg agtggacgga ggaggcgggg gagcgggccg ccctttccga gaggctcttc 3240
   gccaacctgt gggggaggct tgagggggag gagaggctcc tttggcttta ccgggaggtg 3300
   gagaggcccc tttccgctgt cctggcccac atggaggcca cgggggtgcg cctggacgtg 3360
   gcctatctca gggccttgtc cctggaggtg gccgaggaga tcgcccgcct cgaggccgag 3420
   gtcttccgcc tggccggcca ccccttcaac ctcaactccc gggaccagct ggaaagggtc 3480
   ctctttgacg agctagggct tcccgccatc ggcaagacgg agaagaccgg caagcgctcc 3540
   accagcgccg ccgtcctgga ggccctccgc gaggcccacc ccatcgtgga gaagatcctg 3600
   cagtaccggg agctcaccaa gctgaagagc acctacattg accccttgcc ggacctcatc 3660
   caccccagga cgggccgcct ccacacccgc ttcaaccaga cggccacggc cacgggcagg 3720
   ctaagtagct ccgatcccaa cctccagaac atccccgtcc gcaccccgct tgggcagagg 3780
   atccgccggg ccttcatcgc cgaggagggg tggctattgg tggccctgga ctatagccag 3840
   atagagctca gggtgctggc ccacctctcc ggcgacgaga acctgatccg ggtcttccag 3900
   gaggggcggg acatccacac ggagaccgcc agctggatgt tcggcgtccc ccgggaggcc 3960
   gtggaccccc tgatgcgccg ggcggccaag accatcaact tcggggtcct ctacggcatg 4020
   tcggcccacc gcctctccca ggagctagcc atcccttacg aggaggccca ggccttcatt 4080
   gagcgctact ttcagagctt ccccaaggtg cgggcctgga ttgagaagac cctggaggag 4140
   ggcaggaggc gggggtacgt ggagaccctc ttcggccgcc gccgctacgt gccagaccta 4200
   gaggcccggg tgaagagcgt gcgggaggcg gccgagcgca tggccttcaa catgcccgtc 4260
   cagggcaccg ccgccgacct catgaagctg gctatggtga agctcttccc caggctggag 4320
   gaaatggggg ccaggatgct ccttcaggtc cacgacgagc tggtcctcga ggccccaaaa 4380
   gagagggcgg aggccgtggc ccggctggcc aaggaggtca tggagggggt gtatcccctg 4440
   gccgtgcccc tggaggtgga ggtggggata ggggaggact ggctctccgc caaggagtga 4500
   tagatcctct agagtcgacc tgcaggcatg caagcttggc actggccgtc gttttacaac 4560
   gtcgtgactg ggaaaaccct ggcgttaccc aacttaatcg ccttgcagca catccccctt 4620
   tcgccagctg gcgtaatagc gaagaggccc gcaccgatcg cccttcccaa cagttgcgca 4680
   gcctgaatgg cgaatggcgc ctgatgcggt attttctcct tacgcatctg tgcggtattt 4740
   cacaccgcat aaattccctg ttttggcgga tgagagaaga ttttcagcct gatacagatt 4800
   aaatcagaac gcagaagcgg tctgataaaa cagaatttgc ctggcggcag tagcgcggtg 4860
   gtcccacctg accccatgcc gaactcagaa gtgaaacgcc gtagcgccga tggtagtgtg 4920
   gggtctcccc atgcgagagt agggaactgc caggcatcaa ataaaacgaa aggctcagtc 4980
   gaaagactgg gcctttcgtt ttatctgttg tttgtcggtg aacgctctcc tgagtaggac 5040
   aaatccgccg ggagcggatt tgaacgttgc gaagcaacgg cccggagggt ggcgggcagg 5100
   acgcccgcca taaactgcca ggcatcaaat taagcagaag gccatcctga cggatggcct 5160
   ttttgcgttt ctacaaactc ttcctgtcgt catatctaca agccatcccc ccacagatac 5220
   ggtaaactag cctcgttttt gcatcaggaa agcagggaat ttatggtgca ctctcagtac 5280
   aatctgctct gatgccgcat agttaagcca gccccgacac ccgccaacac ccgctgacgc 5340
   gccctgacgg gcttgtctgc tcccggcatc cgcttacaga caagctgtga ccgtctccgg 5400
   gagctgcatg tgtcagaggt tttcaccgtc atcaccgaaa cgcgcgagac gaaagggcct 5460
   cgtgatacgc ctatttttat aggttaatgt catgataata atggtttctt agacgtgagg 5520
   ttctgtaccc gacaccatcg aatggtgcaa aacctttcgc ggtatggcat gatagcgccc 5580
   ggaagagagt caattcaggg tggtgaatgt gaaaccagta acgttatacg atgtcgcaga 5640
   gtatgccggt gtctcttatc agaccgtttc ccgcgtggtg aaccaggcca gccacgtttc 5700
   tgcgaaaacg cgggaaaaag tggaagcggc gatggcggag ctgaattaca ttcccaaccg 5760
   cgtggcacaa caactggcgg gcaaacagtc gttgctgatt ggcgttgcca cctccagtct 5820
   ggccctgcac gcgccgtcgc aaattgtcgc ggcgattaaa tctcgcgccg atcaactggg 5880
   tgccagcgtg gtggtgtcga tggtagaacg aagcggcgtc gaagcctgta aagcggcggt 5940
   gcacaatctt ctcgcgcaac gcgtcagtgg gctgatcatt aactatccgc tggatgacca 6000
   ggatgccatt gctgtggaag ctgcctgcac taatgttccg gcgttatttc ttgatgtctc 6060
   tgaccagaca cccatcaaca gtattatttt ctcccatgaa gacggtacgc gactgggcgt 6120
   ggagcatctg gtcgcattgg gtcaccagca aatcgcgctg ttagcgggcc cattaagttc 6180
   tgtctcggcg cgtctgcgtc tggctggctg gcataaatat ctcactcgca atcaaattca 6240
   gccgatagcg gaacgggaag gcgactggag tgccatgtcc ggttttcaac aaaccatgca 6300
   aatgctgaat gagggcatcg ttcccactgc gatgctggtt gccaacgatc agatggcgct 6360
   gggcgcaatg cgcgccatta ccgagtccgg gctgcgcgtt ggtgcggata tctcggtagt 6420
   gggatacgac gataccgaag acagctcatg ttatatcccg ccgttaacca ccatcaaaca 6480
   ggattttcgc ctgctggggc aaaccagcgt ggaccgcttg ctgcaactct ctcagggcca 6540
   ggcggtgaag ggcaatcagc tgttgcccgt ctcactggtg aaaagaaaaa ccaccctggc 6600
   gcccaatacg caaaccgcct ctccccgcgc gttggccgat tcattaatgc agctggcacg 6660
   acaggtttcc cgactggaaa gcgggcagtg agcgcaacgc aattaatgta agttagctca 6720
   ctcattaggc accccaggct ttacacttta tgcttccgac ctgcaagaac ctcacgtcag 6780
   gtggcacttt tcggggaaat gtgcgcggaa cccctatttg tttatttttc taaatacatt 6840
   caaatatgta tccgctcatg agacaataac cctgataaat gcttcaataa tattgaaaaa 6900
   ggaagagtat gagtattcaa catttccgtg tcgcccttat tccctttttt gcggcatttt 6960
   gccttcctgt ttttgctcac ccagaaacgc tggtgaaagt aaaagatgct gaagatcagt 7020
   tgggtgcacg agtgggttac atc                                         7043
<210> 26
   <211> 10534
   <212> DNA
   <213> Künstliche Sequenz
<220>
   <223> Beschreibung der künstlichen Sequenz: pQE30
<400> 26
   ctcgagaaat cataaaaaat ttatttgctt tgtgagcgga taacaattat aatagattca 60
   attgtgagcg gataagagct ctttagtatt ttttaaataa acgaaacact cgcctattgt 120
   taatattatc taagttaaca ctcgcctatt caatttcaca cagaattcat taaagaggag 180
   aaattaacta tgagaggatc gcatcaccat caccatcacg gatccgttaa agtgtgtctt 240
   aagtaatttc tcctctttaa ttgatactct cctagcgtag tggtagtggt agtgcctagg 300
   acggcaacgg tgatttctta tgacaactac gtcaccatcc ttgatgaaga aacactgaaa 360
   gcgtggattg cgaagtgccg ttgccactaa agaatactgt tgatgcagtg gtaggaacta 420
   cttctttgtg actttcgcac ctaacgcttc ctggaaaaag cgccggtatt tgcatttgat 480
   accgaaaccg acagccttga taacatctct gctaacctgg tcggggacct ttttcgcggc 540
   cataaacgta aactatggct ttggctgtcg gaactattgt agagacgatt ggaccagccc 600
   ctttcttttg ctatcgagcc aggcgtagcg gcatatattc cggttgctca tgattatctt 660
   gatgcgcccg atcaagaaag aaaacgatag ctcggtccgc atcgccgtat ataaggccaa 720
   cgagtactaa tagaactacg cgggctagtt atctctcgcg agcgtgcact cgagttgcta 780
   aaaccgctgc tggaagatga aaaggcgctg aaggtcgggc aaaactagag agcgctcgca 840
   cgtgagctca acgattttgg cgacgacctt ctacttttcc gcgacttcca gcccgttttg 900
   ctgaaatacg atcgcggtat tctggcgaac tacggcattg aactgcgtgg gattgcgttt 960
   gataccatgc tggaggactt tatgctagcg ccataagacc gcttgatgcc gtaacttgac 1020
   gcaccctaac gcaaactatg gtacgacctc tcctacattc tcaatagcgt tgccgggcgt 1080
   cacgatatgg acagcctcgc ggaacgttgg ttgaagcaca aaaccaggat gtaagagtta 1140
   tcgcaacggc ccgcagtgct atacctgtcg gagcgccttg caaccaactt cgtgttttgg 1200
   atcacttttg aagagattgc tggtaaaggc aaaaatcaac tgacctttaa ccagattgcc 1260
   ctcgaagaag ccggatagtg aaaacttctc taacgaccat ttccgttttt agttgactgg 1320
   aaattggtct aacgggagct tcttcggcct cgttacgccg ccgaagatgc agatgtcacc 1380
   ttgcagttgc atctgaaaat gtggccggat ctgcaaaaac acaaagcaat gcggcggctt 1440
   ctacgtctac agtggaacgt caacgtagac ttttacaccg gcctagacgt ttttgtgttt 1500
   gggccgttga acgtcttcga gaatatcgaa atgccgctgg tgccggtgct ttcacgcatt 1560
   gaacgtaacg gtgtgcccgg caacttgcag aagctcttat agctttacgg cgaccacggc 1620
   cacgaaagtg cgtaacttgc attgccacac aagatcgatc cgaaagtgct gcacaatcat 1680
   tctgaagagc tcacccttcg tctggctgag ctggaaaaga aagcgttcta gctaggcttt 1740
   cacgacgtgt tagtaagact tctcgagtgg gaagcagacc gactcgacct tttctttcgc 1800
   catgaaattg caggtgagga atttaacctt tcttccacca agcagttaca aaccattctc 1860
   tttgaaaaac agggcgtact ttaacgtcca ctccttaaat tggaaagaag gtggttcgtc 1920
   aatgtttggt aagagaaact ttttgtcccg attaaaccgc tgaagaaaac gccgggtggc 1980
   gcgccgtcaa cgtcggaaga ggtactggaa gaactggcgc tggactaatt tggcgacttc 2040
   ttttgcggcc caccgcgcgg cagttgcagc cttctccatg accttcttga ccgcgacctg 2100
   tatccgttgc caaaagtgat tctggagtat cgtggtctgg cgaagctgaa atcgacctac 2160
   accgacaagc tgccgatagg caacggtttt cactaagacc tcatagcacc agaccgcttc 2220
   gactttagct ggatgtggct gttcgacggc ctgatgatca acccgaaaac cgggcgtgtg 2280
   catacctctt atcaccaggc agtaactgca acgggacgtt tatcggacta ctagttgggc 2340
   ttttggcccg cacacgtatg gagaatagtg gtccgtcatt gacgttgccc tgcaaatagc 2400
   tcaaccgatc ctaacctgca aaacattccg gtgcgtaacg aagaaggtcg tcgtatccgc 2460
   caggcgttta ttgcgagttg gctaggattg gacgttttgt aaggccacgc attgcttctt 2520
   ccagcagcat aggcggtccg caaataacgc ccagaggatt atgtgattgt ctcagcggac 2580
   tactcgcaga ttgaactgcg cattatggcg catctttcgc gtgacggtct cctaatacac 2640
   taacagagtc gcctgatgag cgtctaactt gacgcgtaat accgcgtaga aagcgcactg 2700
   aaaggcttgc tgaccgcatt cgcggaagga aaagatatcc accgggcaac ggcggcagaa 2760
   gtgtttggtt tgccatttcc gaacgactgg cgtaagcgcc ttccttttct ataggtggcc 2820
   cgttgccgcc gtcttcacaa accaaacggt ctggaaaccg tcaccagcga gcaacgccgt 2880
   agcgcgaaag cgatcaactt tggtctgatt tatggcatga gtgctgacct ttggcagtgg 2940
   tcgctcgttg cggcatcgcg ctttcgctag ttgaaaccag actaaatacc gtactcacga 3000
   ttcggtctgg cgcggcaatt gaacattcca cgtaaagaag cgcagaagta catggacctt 3060
   tacttcgaac gctacaagcc agaccgcgcc gttaacttgt aaggtgcatt tcttcgcgtc 3120
   ttcatgtacc tggaaatgaa gcttgcgatg cctggcgtgc tggagtatat ggaacgcacc 3180
   cgtgctcagg cgaaagagca gggctacgtt gaaacgctgg acggaggacc gcacgacctc 3240
   atataccttg cgtgggcacg agtccgcttt ctcgtcccga tgcaactttg cgacctgcct 3300
   cgccgtctgt atctgccgga tatcaaatcc agcaatggtg ctcgtcgtgc agcggctgaa 3360
   cgtgcagcca ttaacgcggc agacatagac ggcctatagt ttaggtcgtt accacgagca 3420
   gcacgtcgcc gacttgcacg tcggtaattg gcgccaatgc agggaaccgc cgccgacatt 3480
   atcaaacggg cgatgattgc cgttgatgcg tggttacagg ctgagcgcgg ttacgtccct 3540
   tggcggcggc tgtaatagtt tgcccgctac taacggcaac tacgcaccaa tgtccgactc 3600
   caaccgcgtg tacgtatgat catgcaggta cacgatgaac tggtatttga agttcataaa 3660
   gatgatgttg atgccgttgg cgcacatgca tactagtacg tccatgtgct acttgaccat 3720
   aaacttcaag tatttctact acaactacgg gtcgcgaagc agattcatca actgatggaa 3780
   aactgtaccc gtctggatgt gccgttgctg gtggaagtgg ggagtcagcg cttcgtctaa 3840
   gtagttgact accttttgac atgggcagac ctacacggca acgaccacct tcacccctca 3900
   ggcgaaaact gggatcaggc gcactaagat tcgcctgcag ccaagcttaa ttagctgagc 3960
   ttggactcct gttgaccgct tttgacccta gtccgcgtga ttctaagcgg acgtcggttc 4020
   gaattaatcg actcgaacct gaggacaact tagatccagt aatgacctca gaactccatc 4080
   tggatttgtt cagaacgctc ggttgccgcc gggcgttttt tattgatcta ggtcattact 4140
   ggagtcttga ggtagaccta aacaagtctt gcgagccaac ggcggcccgc aaaaaataac 4200
   gtgagaatcc aagctagctt ggcgagattt tcaggagcta aggaagctaa aatggagaaa 4260
   aaaatcactg gatatcactc ttaggttcga tcgaaccgct ctaaaagtcc tcgattcctt 4320
   cgattttacc tcttttttta gtgacctata accaccgttg atatatccca atggcatcgt 4380
   aaagaacatt ttgaggcatt tcagtcagtt gctcaatgta cctattggtg gcaactatat 4440
   agggttaccg tagcatttct tgtaaaactc cgtaaagtca gtcaacgagt tacatggata 4500
   aaccagaccg ttcagctgga tattacggcc tttttaaaga ccgtaaagaa aaataagcac 4560
   aagttttatc cggccttggt ctggcaagtc gacctataat gccggaaaaa tttctggcat 4620
   ttctttttat tcgtgttcaa aataggccgg tttattcaca ttcttgcccg cctgatgaat 4680
   gctcatccgg aatttcgtat ggcaatgaaa gacggtgagc tggtgaaata agtgtaagaa 4740
   cgggcggact acttacgagt aggccttaaa gcataccgtt actttctgcc actcgaccac 4800
   atatgggata gtgttcaccc ttgttacacc gttttccatg agcaaactga aacgttttca 4860
   tcgctctgga gtgaatatac cctatcacaa gtgggaacaa tgtggcaaaa ggtactcgtt 4920
   tgactttgca aaagtagcga gacctcactt taccacgacg atttccggca gtttctacac 4980
   atatattcgc aagatgtggc gtgttacggt gaaaacctgg cctatatggt gctgctaaag 5040
   gccgtcaaag atgtgtatat aagcgttcta caccgcacaa tgccactttt ggaccggata 5100
   ttccctaaag ggtttattga gaatatgttt ttcgtctcag ccaatccctg ggtgagtttc 5160
   accagttttg atttaaaggg atttcccaaa taactcttat acaaaaagca gagtcggtta 5220
   gggacccact caaagtggtc aaaactaaat aacgtggcca atatggacaa cttcttcgcc 5280
   cccgttttca ccatgggcaa atattatacg caaggcgaca aggtgttgca ccggttatac 5340
   ctgttgaaga agcgggggca aaagtggtac ccgtttataa tatgcgttcc gctgttccac 5400
   ctgatgccgc tggcgattca ggttcatcat gccgtttgtg atggcttcca tgtcggcaga 5460
   atgcttaatg aattagacta cggcgaccgc taagtccaag tagtacggca aacactaccg 5520
   aaggtacagc cgtcttacga attacttaat caacagtact gcgatgagtg gcagggcggg 5580
   gcgtaatttt tttaaggcag ttattggtgc ccttaaacgc ctggggttgt catgacgcta 5640
   ctcaccgtcc cgccccgcat taaaaaaatt ccgtcaataa ccacgggaat ttgcggaccc 5700
   gtaatgactc tctagcttga ggcatcaaat aaaacgaaag gctcagtcga aagactgggc 5760
   ctttcgtttt atctgcatta ctgagagatc gaactccgta gtttattttg ctttccgagt 5820
   cagctttctg acccggaaag caaaatagac ttgtttgtcg gtgaacgctc tcctgagtag 5880
   gacaaatccg ccctctagag ctgcctcgcg cgtttcggtg atgacaacaa acagccactt 5940
   gcgagaggac tcatcctgtt taggcgggag atctcgacgg agcgcgcaaa gccactactg 6000
   ggtgaaaacc tctgacacat gcagctcccg gagacggtca cagcttgtct gtaagcggat 6060
   gccgggagca gacaaccact tttggagact gtgtacgtcg agggcctctg ccagtgtcga 6120
   acagacattc gcctacggcc ctcgtctgtt gcccgtcagg gcgcgtcagc gggtgttggc 6180
   gggtgtcggg gcgcagccat gacccagtca cgtagcgata gcggacgggc agtcccgcgc 6240
   agtcgcccac aaccgcccac agccccgcgt cggtactggg tcagtgcatc gctatcgcct 6300
   gtgtatactg gcttaactat gcggcatcag agcagattgt actgagagtg caccatatgc 6360
   ggtgtgaaat accgccacat atgaccgaat tgatacgccg tagtctcgtc taacatgact 6420
   ctcacgtggt atacgccaca ctttatggcg acagatgcgt aaggagaaaa taccgcatca 6480
   ggcgctcttc cgcttcctcg ctcactgact cgctgcgctc ggtcgtgtct acgcattcct 6540
   cttttatggc gtagtccgcg agaaggcgaa ggagcgagtg actgagcgac gcgagccagc 6600
   ttcggctgcg gcgagcggta tcagctcact caaaggcggt aatacggtta tccacagaat 6660
   caggggataa cgcagaagcc gacgccgctc gccatagtcg agtgagtttc cgccattatg 6720
   ccaataggtg tcttagtccc ctattgcgtc gaaagaacat gtgagcaaaa ggccagcaaa 6780
   aggccaggaa ccgtaaaaag gccgcgttgc tggcgttttt ccatactttc ttgtacactc 6840
   gttttccggt cgttttccgg tccttggcat ttttccggcg caacgaccgc aaaaaggtat 6900
   ggctccgccc ccctgacgag catcacaaaa atcgacgctc aagtcagagg tggcgaaacc 6960
   cgacaggact ataaaccgag gcggggggac tgctcgtagt gtttttagct gcgagttcag 7020
   tctccaccgc tttgggctgt cctgatattt gataccaggc gtttccccct ggaagctccc 7080
   tcgtgcgctc tcctgttccg accctgccgc ttaccggata cctgtctatg gtccgcaaag 7140
   ggggaccttc gagggagcac gcgagaggac aaggctggga cggcgaatgg cctatggaca 7200
   ccgcctttct cccttcggga agcgtggcgc tttctcatag ctcacgctgt aggtatctca 7260
   gttcggtgta ggtcgggcgg aaagagggaa gcccttcgca ccgcgaaaga gtatcgagtg 7320
   cgacatccat agagtcaagc cacatccagc ttcgctccaa gctgggctgt gtgcacgaac 7380
   cccccgttca gcccgaccgc tgcgccttat ccggtaacta tcgtcaagcg aggttcgacc 7440
   cgacacacgt gcttgggggg caagtcgggc tggcgacgcg gaataggcca ttgatagcag 7500
   ttgagtccaa cccggtaaga cacgacttat cgccactggc agcagccact ggtaacagga 7560
   ttagcagagc gaggtaactc aggttgggcc attctgtgct gaatagcggt gaccgtcgtc 7620
   ggtgaccatt gtcctaatcg tctcgctcca atgtaggcgg tgctacagag ttcttgaagt 7680
   ggtggcctaa ctacggctac actagaagga cagtatttgg tatcttacat ccgccacgat 7740
   gtctcaagaa cttcaccacc ggattgatgc cgatgtgatc ttcctgtcat aaaccataga 7800
   gcgctctgct gaagccagtt accttcggaa aaagagttgg tagctcttga tccggcaaac 7860
   aaaccaccgc tggtacgcga gacgacttcg gtcaatggaa gcctttttct caaccatcga 7920
   gaactaggcc gtttgtttgg tggcgaccat gcggtggttt ttttgtttgc aagcagcaga 7980
   ttacgcgcag aaaaaaagga tctcaagaag atcctttgat cttttcgcca ccaaaaaaac 8040
   aaacgttcgt cgtctaatgc gcgtcttttt ttcctagagt tcttctagga aactagaaaa 8100
   ctacggggtc tgacgctcag tggaacgaaa actcacgtta agggattttg gtcatgagat 8160
   tatcaaaaag gatctgatgc cccagactgc gagtcacctt gcttttgagt gcaattccct 8220
   aaaaccagta ctctaatagt ttttcctaga tcacctagat ccttttaaat taaaaatgaa 8280
   gttttaaatc aatctaaagt atatatgagt aaacttggtc tgacaagtgg atctaggaaa 8340
   atttaatttt tacttcaaaa tttagttaga tttcatatat actcatttga accagactgt 8400
   gttaccaatg cttaatcagt gaggcaccta tctcagcgat ctgtctattt cgttcatcca 8460
   tagttgcctg actcccaatg gttacgaatt agtcactccg tggatagagt cgctagacag 8520
   ataaagcaag taggtatcaa cggactgagg ccgtcgtgta gataactacg atacgggagg 8580
   gcttaccatc tggccccagt gctgcaatga taccgcgaga cccacggcag cacatctatt 8640
   gatgctatgc cctcccgaat ggtagaccgg ggtcacgacg ttactatggc gctctgggtg 8700
   gctcaccggc tccagattta tcagcaataa accagccagc cggaagggcc gagcgcagaa 8760
   gtggtcctgc aacttcgagt ggccgaggtc taaatagtcg ttatttggtc ggtcggcctt 8820
   cccggctcgc gtcttcacca ggacgttgaa tatccgcctc catccagtct attaattgtt 8880
   gccgggaagc tagagtaagt agttcgccag ttaatagttt gcgcaatagg cggaggtagg 8940
   tcagataatt aacaacggcc cttcgatctc attcatcaag cggtcaatta tcaaacgcgt 9000
   acgttgttgc cattgctaca ggcatcgtgg tgtcacgctc gtcgtttggt atggcttcat 9060
   tcagctccgg ttccctgcaa caacggtaac gatgtccgta gcaccacagt gcgagcagca 9120
   aaccataccg aagtaagtcg aggccaaggg aacgatcaag gcgagttaca tgatccccca 9180
   tgttgtgcaa aaaagcggtt agctccttcg gtcctccgat cgttgttgct agttccgctc 9240
   aatgtactag ggggtacaac acgttttttc gccaatcgag gaagccagga ggctagcaac 9300
   tcagaagtaa gttggccgca gtgttatcac tcatggttat ggcagcactg cataattctc 9360
   ttactgtcat gccatagtct tcattcaacc ggcgtcacaa tagtgagtac caataccgtc 9420
   gtgacgtatt aagagaatga cagtacggta ccgtaagatg cttttctgtg actggtgagt 9480
   actcaaccaa gtcattctga gaatagtgta tgcggcgacc gagttggcat tctacgaaaa 9540
   gacactgacc actcatgagt tggttcagta agactcttat cacatacgcc gctggctcaa 9600
   gctcttgccc ggcgtcaata cgggataata ccgcgccaca tagcagaact ttaaaagtgc 9660
   tcatcattgg aaaaccgaga acgggccgca gttatgccct attatggcgc ggtgtatcgt 9720
   cttgaaattt tcacgagtag taaccttttg gttcttcggg gcgaaaactc tcaaggatct 9780
   taccgctgtt gagatccagt tcgatgtaac ccactcgtgc acccacaaga agccccgctt 9840
   ttgagagttc ctagaatggc gacaactcta ggtcaagcta cattgggtga gcacgtgggt 9900
   actgatcttc agcatctttt actttcacca gcgtttctgg gtgagcaaaa acaggaaggc 9960
   aaaatgccgc aaaaatgact agaagtcgta gaaaatgaaa gtggtcgcaa agacccactc 10020
   gtttttgtcc ttccgtttta cggcgttttt agggaataag ggcgacacgg aaatgttgaa 10080
   tactcatact cttccttttt caatattatt gaagcattta tcaggtccct tattcccgct 10140
   gtgcctttac aacttatgag tatgagaagg aaaaagttat aataacttcg taaatagtcc 10200
   gttattgtct catgagcgga tacatatttg aatgtattta gaaaaataaa caaatagggg 10260
   ttccgcgcac atttccaata acagagtact cgcctatgta taaacttaca taaatctttt 10320
   tatttgttta tccccaaggc gcgtgtaaag cccgaaaagt gccacctgac gtctaagaaa 10380
   ccattattat catgacatta acctataaaa ataggcgtat cacgagggct tttcacggtg 10440
   gactgcagat tctttggtaa taatagtact gtaattggat atttttatcc gcatagtgct 10500
   ggccctttcg tcttcacccg ggaaagcaga agtg                            10534
<210> 27
   <211> 11
   <212> DNA
   <213> Künstliche Sequenz
<220>
   <223> Beschreibung der künstlichen Sequenz: Matrizenausschnitt (SEQ ID NO: 14); w = t, Wildtyp ; w = a, mutante Matrize
<400> 27
   ctaaagwgac a 11
<210> 28
   <211> 11
   <212> DNA
   <213> Künstliche Sequenz
<220>
   <223> Beschreibung der künstlichen Sequenz: Matrizenauschnitt (SEQ ID NO: 16); r = g, Wildtyp; r = a, Mutante
<400> 28
   gtgaatrcaa c 11
<210> 29
   <211> 11
   <212> DNA
   <213> Künstliche Sequenz
<220>
   <223> Beschreibung der künstlichen Sequenz: Matrizenauschnitt (SEQ ID NO: 12); r = t, Wildtyp; r= a, Mutante
<400> 29
   taaggaycgg a 11

## Beschreibung

Die vorliegende Erfindung betrifft DNA-Polymerasen mit einer speziellen Mutation, die eine erhöhter Fehlpaarungs-Diskriminierung aufweisen, deren Herstellung und Verwendung. Die thermostabilen DNA-Polymerasen mit dieser Mutation sind besonders für diagnostische und molekularbiologische Verfahren, z.B. allel-spezifische PCR geeignet.

### Hintergrund der Erfindung

Seit der Vorstellung der ersten menschlichen Genomsequenzen konzentriert sich die Forschung auf die Entdeckung genetischer Unterschiede zwischen den Individuen wie z.B. Einzelbasenmutationen ("single nucleotide polymorphisms" SNP's). Dies ist von Interesse, da zunehmend ersichtlich wird, dass Einzelbasenvariationen im Genom mit unterschiedlicher Arzneimittelverträglichkeit oder Prädisposition für verschiedenste Krankheiten verknüpft sind. In Zukunft könnte die Kenntnis medizinisch relevanter Nukleotidvariationen es ermöglichen, Therapien auf die individuelle genetische Ausstattung anzupassen und die Behandlung mit Medikamenten, die ineffektiv sind oder sogar zu Nebenwirkungen führen, könnte verhindert werden (Shi, Expert Rev. Mol. Diagn. 1, 363-365 (2001)). Es ist offensichtlich, dass Entwicklungen, die eine zeit- und kosteneffiziente Identifizierung von Nukleotidvariationen ermöglichen, zu weiteren Fortschritten in der Pharmakogenetik führen.
SNPs machen den größten Teil der genetischen Variationen im menschlichen Genom aus, und sind die Ursache für über 90% der Unterschiede zwischen Individuen (Kwok, Annu. Rev. Genomics Hum. Genet 2, 235-258 (2001); Kwok und Chen, Curr. Issues Mol. Biol. 5, 43-60 (2003); Twyman and Primrose, Pharmacogenomics 4, 67-79 (2003)). Zum Nachweis solcher genetischer Variationen und anderer Nukleinsäure-Varianten wie Mutationen können verschiedene Verfahren eingesetzt werden. Beispielsweise kann die Identifikation der Variante einer Target-Nukleinsäure durch Hybridisation der zu analysierenden Nukleinsäure-Probe mit einer Sequenzvarianten -spezifischen Hybridisationssonde unter geeigneten Hybridisationsbedingungen erfolgen (Guo et al., Nat. Biotechnol. 15, 331-335 (1997)).

Es hat sich jedoch herausgestellt, dass derartige Hybridisierungsmethoden insbesondere den klinischen Anforderungen hinsichtlich der erforderlichen Sensitivität derartiger Assays nicht genügen. Deshalb hat zum Nachweis von Mutationen, Single-Nukleotid-Polymorphismen (SNP) sowie anderen allelischen Sequenzvarianten insbesondere auch die PCR (Saiki et al., Science 239, 487-490 (1988)) breiten Eingang in molekularbiologische und diagnostische Untersuchungsverfahren gefunden, wobei eine im Hinblick auf die Existenz einer Variante zu untersuchende Target-Nukleinsäure durch eine Polymerase-Kettenreaktion vor der Hybridisierung amplifiziert wird. Als Hybridisationssonden für derartige Assays werden in der Regel einzelsträngige Oligonukleotide verwendet. Eine abgewandelte Ausführungsform dieser Assays sind solche, die fluoreszierende Hybridationssonden einsetzen (Livak, Genet Anal. 14, 143-149 (1999)). Generell wird angestrebt, Verfahren zur Bestimmung SNPs und andern Sequenzvariationen zu automatisieren (Gut, Hum. Mutat. 17, 475-492 (2001)).
Eine bereits im Stand der Technik bekannte Alternative zur Sequenzvarianten-spezifischen Hybridisierung bietet die sogenannte Allel-spezifische Amplifikation (Newton et al., Nucleic. Acids Res. 17, 2503-2516 (1989); Germer et al., genome res. 10, 258-266 (2000); Gibbs et al., Nucleic. Acids Res. 17, 2437-2448 (1989); Wu et al., PNAS 86, 2757-2769 (1989); Ishikawa et al., Hum. Immunol. 42, 315-318 (1995)). Bei diesem Nachweisverfahren werden bereits während der Amplifikation Varianten-spezifische Amplifikationsprimer eingesetzt, die in der Regel am 3'-terminalen Ende des Primers einen sog. diskriminierenden terminalen Nukleotidrest besitzen, welcher lediglich komplementär zu nur einer speziellen Variante der nachzuweisenden Target-Nukleinsäure ist. Bei dieser Methode werden Nukleotidvariationen durch die An- oder Abwesenheit von DNA-Produkt nach der PCR-Amplifikation bestimmt. Das Prinzip der Allel-spezifischen Amplifikation basiert auf der Ausbildung von kanonischen oder nicht kanonischen Primer-Template-Komplexen am Ende von allel-spezifischen Primersonden. An einem korrekt gepaarten 3'-Primerende kann die Amplifikation durch eine DNA-Polymerase stattfinden, bei einem fehlgepaarten Primerende hingegen sollte die Verlängerung gehemmt sein.
US 5,595,890 beschreibt beispielsweise derartige Verfahren zur Allel-spezifischen Amplifikation sowie deren Anwendung zum Nachweis von klinisch relevanten Punktmutationen, beispielsweise im k-ras-Onkogen. US 5,521,301 beschreibt ebenfalls Verfahren zur Allel-spezifischen Amplifikation zur Genotypisierung des ABO-Blutgruppensystems. US 5,639,611 offenbart dagegen die Verwendung Allel-spezifischer Amplifikation im Zusammenhang mit dem Nachweis der für Sichelzell-Anämie verantwortlichen Punktmutation.
Die Allel-spezifische Amplifikation ist jedoch insofern problematisch, das sie sich durch eine nur geringe Selektivität aufzeichnet, wodurch weitere, aufwendige und damit zeit- und kostenintensive Optimierungsschritte bedingt werden. Derartige Verfahren zum Nachweis von Sequenzvarianten, Polymorphismen und vor allem Punktmutationen erfordern insbesondere dann eine Allel-spezifische Amplifikation, wenn sich die nachzuweisende Sequenzvariante im Unterschuss verglichen mit einer im Überschuss vorhandenen Variante desselben Nukleinsäureabschnitts (bzw. desselben Gens) befindet.
Eine derartige Situation ist beispielsweise dann gegeben, wenn mit Hilfe von Allel-spezifischer Amplifikation disseminierte Tumorzellen in Körperflüssigkeiten wie Blut, Serum oder Plasma nachgewiesen werden sollen (US 5,496,699). Zu diesem Zweck wird zunächst DNA aus Körperflüssigkeiten wie Blut, Serum oder Plasma isoliert, welche sich aus einem Unterschuss DNA aus disseminierten Tumorzellen sowie einem Überschuss an DNA aus nicht proliferierenden Zellen zusammensetzt. Die für die tumorale DNA signifikanten Mutationen im k-Ras Gen müssen somit aufgrund von wenigen Kopien tumoraler DNA in Gegenwart eines Überschusses an Wildtyp DNA nachgewiesen werden.

Alle im Stand der Technik beschriebenen Verfahren zur Allel-spezifischen Amplifikation haben den Nachteil, dass trotz der Verwendung von 3' diskriminierenden Nukleotidresten eine Primerextension in Gegenwart einer geeigneten DNA Polymerase in geringerem Umfang auch dann erfolgt, wenn die Target-Nukleinsäure nicht exakt der nachzuweisenden Sequenzvariante entspricht, d. h., sich von dieser zumindest in dem zum diskriminierenden Nukleotidrest komplementären Nukleotid unterscheidet. Dies führt insbesondere dann zu falsch-positiven Ergebnissen, wenn eine bestimmte Sequenzvariante in einem Überschuß-Background von Nukleinsäuren enthaltend eine andere Sequenzvariante nachgewiesen werden soll. Dies ist wie oben erwähnt beispielsweise bei der Detektion von bestimmten k-Ras-Allelen als Indikator für disseminierte Tumorzellen der Fall. Ein weiterer Nachteil der bekannten Verfahren besteht darin, daß in jedem Falle ein 3' terminal diskriminierender Oligonukleotidrest verwendet werden muß. Maßgeblich für die Nachteile dieser auf PCR basierenden Verfahren ist die Unfähigkeit der in diesen Verfahren eingesetzten Polymerasen, ausreichend zwischen Basenfehlpaarungen zu diskriminieren. Bislang war es daher nicht möglich, direkt durch PCR eine eindeutige Aussage über die An- bzw. Abwesenheit einer Mutation zu machen. Es bedarf bislang immer weiterer zeit- und kostenintensive Aufreinigungs- und Analyseverfahren für eine eindeutige Diagnose solcher Mutationen. Daher werden Neuerungen, die eine Erhöhung der Selektivität der Allel-spezifischen PCR Amplifikation ermöglichen, einen signifikanten Einfluss auf Verlässlichkeit und Robustheit der direkten SNP-Analyse durch die PCR haben.

Andererseits werden bereits eine Reihe von Modifikationen in der Proteinsequenz der DNA-Polymerasen I beschrieben. So erwähnt das US-Patent 6,329,178 DNA-Polymerasemutanten mit geänderter katalytischer Aktivität, bei denen Mutationen in dem A-Motiv (der hochkonservierten Sequenz DYSQIELR) erfolgt waren. Darüber hinaus beschreibt Minnick, T. et al., J. Biol. Chem. 274, 3067 - 3075 (1999) eine Vielzahl von *E.-coli*-DNA-Polymerase-I-(Klenow-Fragment)-Mutanten, bei denen Alaninaustausche vorgenommen wurden. Ein Teil der beschriebenen Mutanten zeigt eine höhere Polymerase-Genauigkeit, bezogen auf den Wildtyp. Eine der erwähnten Mutanten ist H881A; besondere Eigenschaften dieser Mutanten in Bezug auf die anderen beschriebenen Mutanten werden nicht aufgeführt.

Aufgabe der vorliegenden Erfindung war es deshalb, Sequenzvarianten mit erhöhter Spezifität bereitzustellen, mit deren Hilfe ein Sequenzvarianten-spezifisches Nachweisverfahren ermöglicht wird.

### Kurzbeschreibung der Erfindung

Es wurde überraschenderweise gefunden, dass spezielle Mutanten von DNA-Polymerasen der Familie A, nämlich solche, in denen das konservierte C-Motiv und insbesondere dessen QHV-Aminosäuresequenz modifiziert wurde, eine erhöhte Fehlerpaarungs-Diskriminierung aufweisen und in Nachweisverfahren für Sequenzvarianten einsetzbar sind. Die thermostabilen Varianten hiervon sind zur allelspezifischen PCR geeignet. Die vorliegende Erfindung betrifft im Einzelnen
(1) eine DNA-Polymerase der Familie A, die eine modifizierte Motiv-C-Sequenz und eine, im Vergleich zu der entsprechenden Wildtyppolymerase, erhöhte Fehlpaarungs-Diskriminierung aufweist, oder ein Klenow-Fragment derselben;
(2) eine bevorzugte DNA-Polymerase von Ausführungsform (1), wobei in der Motiv-C-Sequenz QVH in Position 879-881, bezogen auf das in SEQ ID NO:2 gezeigte Klenow-Fragment der *E. coli* DNA-Polymerase, wenigstens der Aminosäurerest Q879 durch einen lipophilen Aminosäurerest ersetzt ist;
(3) eine DNA-Sequenz, die die DNA-Polymerase oder deren Klenow-Fragment gemäß Ausführungsform (1) oder (2) codiert;
(4) einen Vektor, der die DNA-Sequenz gemäß Ausführungsform (3) enthält;
(5) eine Wirtszelle, die mit dem Vektor gemäß Ausführungsform (4) transformiert ist und/oder eine DNA gemäß Ausführungsform (3) aufweist;
(6) ein Verfahren zur Herstellung einer DNA-Polymerase oder deren Klenow-Fragment gemäß der Ausführungsform (1) oder (2), umfassend das Kultivieren einer Wirtszelle gemäß Ausführungsform (5) und das Isolieren der DNA-Polymerase oder des Klenow-Fragments aus der Kultur oder dem Kulturüberstand;
(7) die Verwendung der DNA-Polymerase oder des Klenow-Fragments gemäß Ausführungsform (1) oder (2) in diagnostischen und molekularbiologischen in-vitro- Vefahren, einschließlich allel-spezifischer PCR, DNA-Ampilfikatibn mittels PCR, Klonierung usw.;
(8) ein Verfahren zur Bestimmung der An- oder Abwesenheit von mindestens einer Sequenzvariante in einer oder mehreren Target-Nukleinsäuren in einer individuellen Probe unter Verwendung einer DNA-Polymerase gemäß der Ausführungsform (1) oder (2); und
(9) einen Kit zur Bestimmung der An- oder Abwesenheit von mindestens einer Sequenzvariante in einer oder mehreren Target-Nukleinsäuren in einer individuellen Probe gemäß des Verfahrens nach Ausführungsform (8) enthaltend wenigstens eine DNA-Polymerase gemäß Ausführungsform (1) oder (2).

### Kurzbeschreibung der Figuren

Fg. 1: Autoradiogramme nach denaturierender PAGE zur Untersuchung des Einflusses von Mutationen in *E. coli* DNA-Polymerase I (Klenow-Fragment, 5'→3'-Exonuklease-defizient) in Positionen 879-881 (SEQ ID NO:2) auf die Selektivität der Primerverlängerung. Reaktionen enthielten 150 nM Primer-/Matrizen-Komplex (Primer: 5'-ACA AAA TAC CTG TAT TCC TX-3', X= A, G, C oder T (SEQ ID NO:11); Matrize: 5'-GAA TCC CTG GAC AGG CYA GGA ATA CAG GTA TTT TGT-3', Y= A, G, C oder T (SEQ ID NO:12), 1 mM jeweils dATP, dCTP, TTP, dGTP und 600 nM DNA-Polymerase. Inkubation bei 37°C für 10 min in Puffer (50 mM Tris-HCl pH 7,3, 10 mM MgCl₂, 1 mM DTT, 0,05% Triton^{®} X-100).

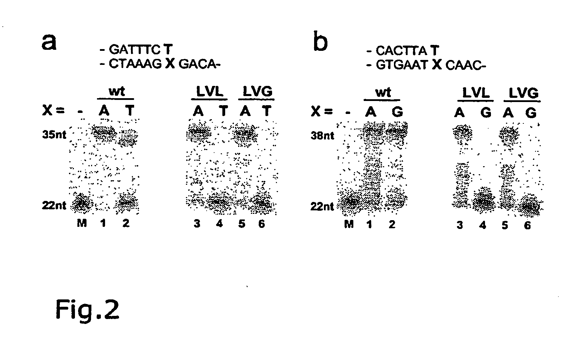

Fig. 2: Autoradiogramme nach denaturierender PAGE zur Untersuchung des Einflusses von Mutationen in *E. coli* DNA-Polymerase I (Klenow-Fragment, 5'→>3'-Exonuklease-defizient) in Positionen 879-881 (bezogen auf das in SEQ ID NO:2 gezeigte Klenow-Fragment aus *E. coli*) auf die Selektivität der Primerverlängerung. Reaktionen enthielten 150 nM Primer-/Matrizen-Komplex [a: Primer: 5'-GAC CCA CTC CAT CGA GAT TTC T-3'(SEQ ID NO:14); Matrizen: 5'-GGA CTA GCT ACA GXG AAA TCT CGA TGG AGT GGG TC-3', X = A oder T (SEQ ID NO:15); b: Primer: 5'-GT TTA GAT GTT AAA TCA CAC TTA T-3' (SEQ ID NO:15); Matrize: 5'-CTT TCC AGA CAA CXT AAG TGT GAT TTA ACA TCT AAA AC-3', X = A oder G (SEQ ID NO:16)], 1 mM jeweils dATP, dCTP, TTP, dGTP und 600 nM DNA-Polymerase. Inkubation bei 37°C für 10 min in Puffer (50mM Tris-HCl pH 7,3, 10 mM MgCl₂, 1 mM DTT, 0,05% Triton^{®} X-100).

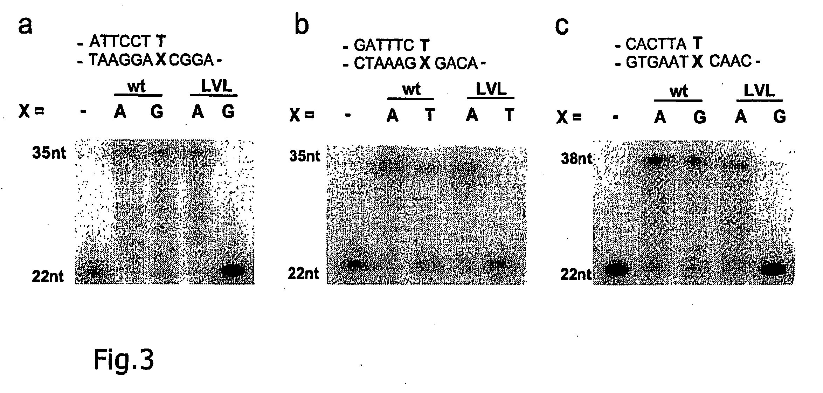

Fig. 3: Autoradiogramme nach denaturierender PAGE zur Untersuchung des Einflusses von Mutationen im QVH-Motiv in Taq DNA-Polymerase I auf die Selektivität der Primerverlängerung. Reaktionen enthielten 150 nM Primer-/Matrizen-Komplex [a: Primer: 5'-ACA AAA TAC CTG TAT TCC TX-3', X= T (SEQ ID NO:11); Matrize: 5'-GA TCC CTG GAC AGG CYA GGA ATA CAG GTA TTT TGT-3', Y = A oder G (SEQ ID NO:12); b: Primer: 5'-GAA CCA CTC CAT CGA GAT TTC T-3' (SEQ ID NO:13); Matrize: 5'-GGT CTA GCT ACA GXG AAA TCT CGA TGG AGT GGG TC-3', X = A oder T (SEQ ID NO:14); c: Primer: 5'-GT TTA GAT GTT AAA TCA CAC TTA T-3' (SEQ ID NO:15); Matrize: 5' -CTT TCC AGA CAA CXT AAG TGT GAT TTA ACA TCT AAA AC-3', X = A oder G (SEQ ID NO:16)], 1mM jeweils dATP, dCTP, TTP, dGTP und 0,6 ng DNA-Polymerase. Inkubation bei 37 °C für 10 min in Puffer (50 mM Tris-HCl (pH 9,2 bei 25°C), 16 mM Ammoniumsulfat, 2,5 mM MgCl₂, 0,1 % Tween^{®} 20).

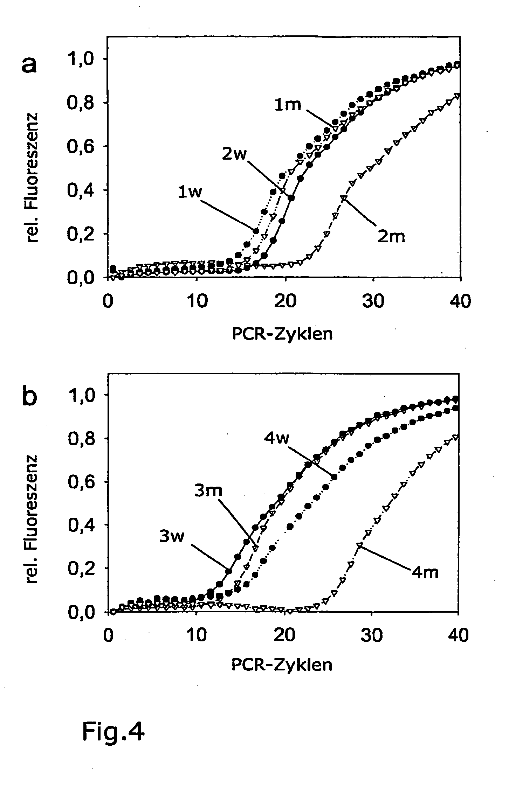

Fig. 4: Real-time PCR Experimente mit Taq (wt) (SEQ ID NO:4) und LVL-Mutante (SEQ ID NO:4 mit LVL in Positionen 782 - 784). Die Experimente wurden mittels einem *iCycler (BIORAD)* System durchgeführt. Typische Reaktion in 20 µl enthielt: 40 pM der betreffenden Matrize in *Taq* DNA-Polymerase Puffer (50 mM Tris-HCl (pH 9,2 bei 25°C), 16 mM Ammoniumsulfat, 2,5 mM MgCl ₂, 0,1% Tween^{®} 20, 0,3 mM dNTPs) 0,5 µM der beiden Primer und 95 ng *Taq* DNA-Polymerase und eine 1/50.000 von *SybrGreen I* 10.000x-Lösung in DMSO (*Molecular Probes*). Die PCR wurde mit folgendem Programm durchgeführt: Zyklen bei 95°C für 30 s, 55°C für 35 s und 72°C für 40 s. Reaktionen 1w, 2w, 3w und 4w wurden mit dem Wildtyp-Enzym, 1m, 2m, 3m und 4m mit der LVL-Mutante durchgeführt. DNA-Sequenzen:
   a: Primersonde: 5'-d(GAC CCA CTC CAT CGA GAT TTC T) (SEQ ID NO:19); Reverse Primer: 5'-d(AGA GGA AAG ATG AAG TAC TAT G) (SEQ ID NO:20); Matrize: 5'-d(CAA CTG TTC AAA CTG ATG GGA CCC ACT CCA TCG AGA TTT CXC TGT AGC TAG ACC AAA ATC ACC TAT TTT TAC TGT GAG GTC TTC ATG AAG AAA TAT ATC TGA GGT GTA GTA AGT AAA GGA AAA CAG TAG ATC TCA TTT TCC TAT CAG AGC AAG CAT TAT GAA GAG TTT AGG TAA GAG ATC TAA TTT CTA TAA TTC TGT AAT ATA ATA TTC TTT AAA ACA TAG TAC TTC ATC TTT CCT CT), X= A (wildtyp)(1) oder T (Mutante)(2) (SEQ ID NO:21).
   b: Primersonde: 5'-d(GTT TTA GAT GT TAA ATC ACA CTT AT) (SEQ ID NO:22); Reverse Primer: 5'-d(AAA GCT CCT TTC TGA ATA TTG AG) (SEQ ID NO:23); Matrize: 5'-d(AAA ATG TGA GAA GGG ACC TCA TAA AAT ATG TCA TAT GGA AAT GAG CAG ATA ATA AAG ATT ATA GCT TTT CTT TGT CAA AAG GAG ACT CAA TAT CTT TAC TCT TTC ATC AGG ACA TTG TGA CAA ATG TTT CCC CCA GAA TCA TCC GGG GAA CCA CCT CTG GCC CCA TGT ATG GCC CTG GAC AAA GCT CCT TTC TGA ATA TTG AGC TCA TCA GTG AGA AAA CGG CTG CAT ATT GGT GTC AAA GTG TCA CTG AAC TAA AGG CTG ACT TTC CAG ACA ACX TAA GTG TGA TTT AAC ATC TAA AAC), X= A (wildtyp)(3) oder G (Mutante)(4) (SEQ ID NO:24).

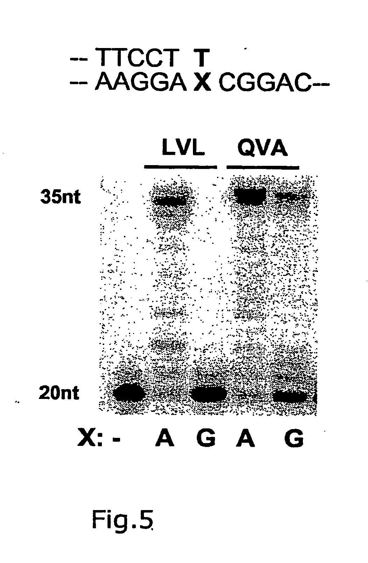

Fig 5: Autoradiogramme nach denaturierender PAGE zur vergleichenden Untersuchung des Einflusses von Mutationen in *E. coli* DNA-Polymerase I (Klenow-Fragment, 5'→3'-Exonuklease-defizient) in Positionen 879-881 (SEQ ID NO:2) auf die Selektivität der Primerverlängerung. Verglichen wurde die erfindungsgemäße Mutante LVL mit der literaturbekannten Mutante QVA (Minnick, T. et al., J. Biol. Chem. 274, 3067 - 3075 (1999)). Reaktionen enthielten 150 nM Primer-/Matrizen-Komplex (Primer: 5'-ACA AAA TAC CTG TAT TCC TX-3', X= A, G, C oder T (SEQ ID NO:11); Matrize: 5'-GA TCC CTG GAC AGG CYA GGA ATA CAG GTA TTT TGT-3', Y= A, G, C oder T (SEQ ID NO:12), 1 mM jeweils dATP, dCTP, TTP, dGTP und 600 nM DNA-Polymerase. Inkubation bei 37°C für 10 min in Puffer (50 mM Tris-HCl pH 7,3, 10 mM MgCl₂, 1 mM DTT, 0,05% Triton^{®} X-100).

### Sequenzprotokoll - Freier Text

| SEQ ID NO: | Beschreibung - freier Text |
|---|---|
| 1 | E. coli Wildtyp Klenow Fragment der DNA Polymerase 1 |
| 2 | E.coli Klenow Fragment der DNA Polymerase 1 |
| 3 | Wildtyp Taq Polymerase |
| 4 | Wildtyp Taq Polymerase |
| 5 | Primer |
| 6 | Downstream-Primer |
| 7 | Antisense-Primer |
| 8 | Primer FVL20TH |
| 9 | Matrize TFVL90A |
| 10 | Matrize TFVL90G |
| 11 | Primer zur Detektion des SNPs in der humanen genomischen Factor-V-Leiden-DNA-Sequenz |
| 12 | Matrize der humanen genomischen Factor-V-Leiden-DNA-Sequenz; n=g, Wildtypmatrize; n=a, mutante Matrize |
| 13 | Primer zur Detektion der humanen somatische BRAF-T1796A-Mutation |
| 14 | Wildtyp Matrize des BRAF Gens; w= t, Wildtypmatrize; w = a, mutante Matrize |
| 15 | Primer zur Detektion der humanen Dihydropyrimidin-Dehydrogenase (DPyD) Mutation G735A |
| 16 | Matrize des humanen DPyD; r = g, Wildtypmatrize; r = a, mutante Matrize |
| 17 | Primer zur Detektion der humanen sauren Ceramidase Mutation A107G |
| 18 | Matrize der humanen sauren Ceramidase; r = a, Wildtypmatrize; r = g, mutante Matrize |
| 19 | Primersonde BrafT |
| 20 | umgekehrter Primer für BRAF |
| 21 | Zielmatrize BrafX; w = a, Braf A (Wildtyp); w = t, BrafT (Mutante) |
| 22 | Primersonde DpyDT |
| 23 | umgekehrter Primer für DpyDT |
| 24 | Zielmatrize DpyDX; r = a, DpyDA (Wildtyp); r = t, DpyDT (Mutante) |
| 25 | pTTQ18::Taq |
| 26 | pQE30 |
| 27 | Matrizenausschnitt (SEQ ID NO:14); w = t, Wildtyp; w = a, mutante Matrize |
| 28 | Matrizenauschnitt (SEQ ID NO:16); r = g, Wildtyp; r = a, Mutante |
| 29 | Matrizenauschnitt (SEQ ID NO:12); r = t, Wildtyp; r= a, Mutante |

### Detaillierte Beschreibung der Erfindung

Gegenstand der vorliegenden Erfindung sind mutierte DNA-Polymerasen der Familie A mit erhöhter Fehlpaarungs-Diskriminierungsfähigkeit, oder Klenow-Fragmente derselben. Die erhöhte Fehlpaarungs-Diskriminierungsfähigkeit - worunter eine hohe Selektivität gemäß der Watson-Crick-Regeln beim Einbau von komplementären Basen, wie auch schon der Anlagerung eines Primers an die Matrize zu verstehen ist, d. h. ein größeres Verlängerungsselektivitätsverhältnis (F_{match}/F_{mismatch}) als die entsprechende Ausgangspolymerase (Wildtyp) aufweist (z. B. bestimmt in dem Fluoreszenz-Testsystem gemäß Beispiel 2) - kann durch Mutation einer bestimmten Aminosäuresequenz in natürlichen Enzymen erreicht werden. Die dadurch bedingten Eigenschaften der DNA-Polymerasen übertreffen die der "State-of-the-art" Wildtyppolymerasen, wie sie derzeit kommerziell vertrieben werden. Die Erhöhung der Selektivität der Aktivität von DNA-Polymerasen ermöglicht verlässlichere Systeme zum Nachweis von Mutationen oder Polymorphismen, die direkte Diagnose durch allel-spezifische PCR ohne nachgeschaltete zeit- und kostenintensive Aufreinigungs- un d Analyseverfahren und hohe Nachhaltigkeit, da keine chemischen modifizierten Primer verwendet werden müssen.
Nachfolgend Definitionen sind auf die gesamte Anmeldung anzuwenden, sind aber nicht limitierend zu verstehen. "DNA-Polymerasen der Familie A" (auch Polymerasen I genannt) sind solche DNA-polymerisierenden Enzyme, die das A-Motiv mit der Sequenz DYSQIELR in ihrem aktiven Zentrum aufweisen. Es zählen dazu auch die hier beschriebenen Enzyme, die Mutationen im C-Motiv aufweisen. Insbesondere zählen zu diesen auch thermostabile DNA Polymerasen, sowie deren Mutanten.
Unter dem Begriff "Klenow-Fragment" wird ein jedes C-terminale Fragment einer DNA-Polymerase der Familie A verstanden, das sowohl Polymeraseaktivität wie auch 3'→5' Exonukleaseaktivität (aber keine 5'→3' Exonukleaseaktivität) besitzt. Unter "Vektoren" im Sinne der vorliegenden Erfindung sind Plasmide, Cosmide, Phagemide und Viren zu verstehen, die neben einer DNA von Interesse (wobei es sich insbesondere um erfindungsgemäße Sequenzen von DNA-Polymerasen aus der Familie A handelt) auch Kontrollelemente, die die Exprimierung der DNA von Interesse steuern, umfassen.
Unter den Begriff "Wirtszellen" fallen sowohl prokaryontische Zellen, wie *E*. *coli* (insbesondere *E. coli* XI1 -blue, DH5α, BI21 (DE3), M15 [pREP4], SG13005 [pREP4], BL21 (DE3) pLysS), *Halomonas elongata, Caulobacter sp., Halobacterium halobium* usw., als auch eukaryontische Zellen, wie Hefe- und andere Pilzzellen, pflanzliche und tierische Zellen, einschließlich isolierter menschlicher Zellen, die sich in Zellkultur befinden. Des Weiteren werden unter dem Begriff "Wirtszelle" auch Zellextrakte verstanden, die bei Vorlage einer mRNA diese translatieren können, wie Weizenkeimextrakt (wheat germ extract) als auch Kaninchen-Reticulocytenextrakt (Rabbit reticulocyte extract, (RRL)). Weiterhin werden hier auch *in vitro* Expressionssystem als "Wirtszelle" verstanden, wie z. B. das T7 Expression System, pBAD Expression System, ThioFusion™ Expression Systems, trc Expression System, PL Expression System, PurePro™ Caulobacter Expression System, Microbiological Media and Media Components, Qiagen pQE Expression System und das Novagen pET Expression System usw.

Ausführungsformen (1) und (2) der Erfindung betreffen DNA-Polymerasen der Familie A oder deren Klenow-Fragment, die sich von natürlich vorkommenden DNA-Polymerasen dadurch unterscheiden, dass sie eine erhöhte Fehlpaarungsdiskriminierung aufweisen, was zu einer erhöhten Selektivität der Enzymaktivität führt. Die erfindungsgemäßen DNA-Polymerasen leiten sich von bakteriellen DNA-Polymerasen, wie Polymerasen von *E. coli, Aquifex, Borielia, Bacillus, Chlamydia, Chlamydophila, Chloroflexus, Haemophilis, Heliobacter, Lacococcus, Methylobakterium, Myocobakterium, Rohodothermus, Rickettsia, Streptococcus, Streptomyces, Syncechocysts, Treponema* usw., insbesondere jedoch auch von Polymerasen thermostabiler Organismen wie *Thermus aquaticus, Thermus thermophilus, Thermus filiformis, Rhodothermus obamensis, Bacillus stearothermophilus* usw. ab. Bei den erfindungsgemäßen DNA-Polymerasen gemäß Ausführungsform (1) oder deren Klenow-Fragmenten ist dabei insbesondere in der Motiv-C-Sequenz QVH in Positionen 879 - 881 (in Bezug auf das in SEQ ID NO:2 gezeigte Klenow-Fragment der DNA-Polymerase aus *E. coli*) wenigstens ein Aminosäurerest, vorzugsweise das Q und/oder das H, durch einen lipophilen Aminosäurerest ersetzt.

In Ausführungsform (2) der Erfindung ist wenigstens der Aminosäurerest Q879 bezogen auf SEQ ID NO:2 durch einen lipophilen Aminosäurerest ersetzt. In einem bevorzugten Aspekt der Ausführungsform (2) ist in der Motiv-C-Sequenz QVH weiterhin der Aminosäurerest H881 durch einen lipophilen Aminosäurerest ersetzt.

In einem weiteren bevorzugten Aspekt der Ausführungsformen (1) und (2) ist der Aminosäurerest in Position 880 (bezogen auf SEQ ID NO:2) ausgewählt aus Val, Leu, Ile, Ala oder Tyr und besonders bevorzugt ausgewählt aus Val und Ile.

"Lipophite Aminosäurereste" im Sinne der vorliegenden Erfindung umfassen die Aminosäurereste Gly, Ala, Val, Leu, Met, Phe, Trp, Ile, Pro usw. Bevorzugte Reste sind dabei Gly, Ala, Val, Leu und Ile. Im Sinne der vorliegenden Erfindung ist die Motiv-C-Sequenz QVH vorzugsweise durch die Sequenzen LVL, LVG, QVL, PIL, QW, LVA, LAA, LW, LVI, IVI, III, VVV, QVV, QVA usw. ersetzt, wobei ein Austausch durch LVL und LVG besonders bevorzugt ist.

Neben dem vorstehend genannten Austausch kann die erfindungsgemäße Polymerase noch weitere Mutationen wie z. B. Deletionen, Substitutionen und/oder Additionen (jeweils bis zu 20 Aminosäureresten) aufweisen, vorausgesetzt dass dadurch das größere Verlängerungsverhältnis (F_{match}/F_{mismatch}), bezogen auf den Wildtyp, nicht beeinträchtigt wird. Zu den Substitutionen zählen dabei insbesondere weitere (vorzugsweise konservative) Austausche in der Motiv-C-Sequenz, die neben dem oben aufgeführten Ersetzen wenigstens eines Restes in QVH durch einen lipophilen Aminosäurerest erfolgen. So umfasst die vorliegende Erfindung insbesondere auch solche DNA-Polymerasen, die eine Aminosäuresequenz LVN, LYH, PLQ, LVQ, QDL, QEL, QUV usw. an der Stelle von QVH im C-Motiv enthalten.
Darüber hinaus wurde gefunden, dass auch eine DNA-Polymerase mit der Sequenz QVN anstelle von QVH im C-Motiv ein größeres Verlängerungsverhältnis (F_{match}/F_{mismatch}), bezogen auf den Wildtyp, aufweist.

Weiterhin beinhaltet die Erfindung eine Taq-Polymerase, deren QVH Sequenz wie vorstehend beschrieben ausgetauscht wurde. Besonders bevorzugt sind dabei solche Taq-Polymerasen, deren QVH Sequenz durch LVL oder LVG ausgetauscht wurde (in Bezug auf die in SEQ ID NO:4 gezeigte Taq Polymerase Proteinsequenz sind die Positionen 782-784 vom Austausch betroffen).

Bei den erfindungsgemäßen Klenow-Fragmenten ist es bevorzugt, dass wenigstens zwei Aminosäurereste in QVH durch lipophile Aminosäurereste ersetzt sind.

Besonders bevorzugt unter den vorstehend genannten Sequenzen mit zwei Austauschen sind - auch für die Klenow-Fragmente - LVL und LVG.

Die erfindungsgemäßen Polymerase-Mutanten besitzen im Vergleich zu der literaturbekannten Mutante QVA (Minnick, T. et al., J. Biol. Chem. 274, 3067-3075 (1999)) eine erhöhte Selektivität der Primerverlängerung (Bsp. 7, Fig. 5). Wie Figur 5 zeigt, hat die QVA-Mutante eine wesentlich höhere Neigung als die LVL-Mutante gemäß der vorliegenden Erfindung, Fehlpaarungen zu verlängern.

Die Aminosäuresequenz der erfindungsgemäßen DNA-Polymerasen wurde somit an einer oder mehreren Stellen verändert im Vergleich zu den DNA-Wildtyppolymerasen, und dies spiegelt sich auch auf Nukleinsäureebene wieder. Erfindungsgegenstand ist auch eine DNA Sequenz, die für eine der erfindungsgemäßen DNA-Polymerasen oder deren Klenow-Fragment codiert (Ausführungsform (3) der Erfindung). Ebenso ist ein Vektor, der die DNA-Polymerase (1) oder (2) codierende DNA Sequenz enthält, sowie eine Wirtszelle, die mit einem Vektor transformiert ist, der die DNA-Polymerase (1) oder (2) codierende DNA Sequenz enthält, und/oder die eine die DNA-Polymerase (1) oder (2) codierende DNA aufweist, Gegenstand der Erfindung. Ebenfalls Erfindungsgegenstand ist ein Verfahren zur Herstellung einer DNA-Polymerase oder deren Klenow-Fragment, welches das Kultivieren der transformierten Wirtszelle und das Isolieren der DNA-Polymerase oder des Klenow-Fragments aus der Kultur oder dem Kulturüberstand umfasst.
Thermostabile DNA-Polymerasen gemäß Ausführungsform (1) oder (2) der Erfindung haben eine höhere Selektivität in der Polymerase Kettenreaktion (PCR) und diskriminieren besser zwischen einzelnen Fehlpaarungen und kanonischen Komplexen als natürlich vorkommende DNA-Polymerasen. Dies bedingt verbesserte Eigenschaften der Mutanten im Einsatz von diagnostischen (Allel-spezifische PCR) und molekularbiologischen (DNA-Amplifikation mittels PCR, Klonierung) Verfahren. Verfahren, in denen die erfindungsgemäße DNA-Polymerase oder deren Klenow-Fragment eingesetzt werden kann, sind daher ebenfalls Gegenstand der Erfindung.

Die DNA-Polymerase gemäß Ausführungsform (1) oder (2) der Erfindung ist auch in einem Verfahren zur Bestimmung der An- oder Abwesenheit von Sequenzvarianten in einer oder mehreren Target-Nukleinsäuren in einer individuellen Probe einsetzbar (Ausführungsform (8)). Solch ein Verfahren umfasst vorzugsweise einen oder mehrere der folgenden Schritte:
a) Zugabe von
   - Desoxy-Nukleosid-Triphosphaten,
   - einer der erfindungsgemäßen DNA-Polymerasen
   - mindestens einem diskriminierenden Primer, enthaltend mindestens einen diskriminierenden Nukleotidrest, wobei für jede nachzuweisende Sequenzvariante einer Target-Nukleinsäure ein Primer zugegeben wird, welcher eine Sequenz komplementär zur nachzuweisenden Sequenzvariante aufweist, und wobei die nachzuweisende Sequenzvariante in der Target-Nukleinsäure komplementär zu mindestens einem 3'-terminalen-, 3'-proxi-terminalen- , oder 3'-proxi-proxi-terminalen Nukleotidrest des diskriminierenden Primers ist,
   - mindestens einem weiteren Primer, der komplementär ist zu einem Primer-Extentsionsprodukt, entstehend durch Extension eines diskriminierenden Primers
b) Durchführung einer Primer-Extensionsreaktion, wobei ein Verlängerungsprodukt des diskriminierenden Primers im Wesentlichen nur dann erhalten wird, wenn die Probe eine Target-Nukleinsäure mit der nachzuweisenden Sequenzvariante enthält
c) Trennung des Produkts der Primer-Extensionsreaktion von der Template-Nukleinsäure
d) Zyklische Wiederholung der Schritte b) und c) zum Erhalt eines Amplifikationsproduktes, beispielsweise durch Polymerase-Kettenreaktion
e) Bestimmmung der An- oder Abwesenheit einer Sequenzvariante aufgrund der An- oder Abwesenheit des Amplifikationsproduktes.

In diesem Zusammenhang sind die für die Beschreibung des erfindungsgemäßen Verfahrens verwendeten Begriffe wie folgt zu verstehen:
Eine "erfindungsgemäße DNA Polymerase" ist dabei eine wie vorstehend definierte DNA-Polymerase der Familie A, die das A-Motiv mit der Sequenz DYSQIELR in ihrem aktiven Zentrum aufweist und bestimmte Mutationen im C-Motiv besitzt. Insbesondere zählen zu diesen auch thermostabile DNA Polymerasen mit Mutationen im C-Motiv. Bei den Mutationen handelt es sich um konservative Substitutionen der QVL Aminosäurereste des C-Motivs und/oder die vorstehend definierten nicht-konservierenden Substitutionen.
Eine "thermostabile DNA Polymerase" ist eine Polymerase, die auch bei Temperaturen von über 42°C funktionsfähig ist, und Insbesondere bei auf PCR basierenden Amplifikationsverfahren eingesetzt werden kann.
Insbesondere fallen unter den Begriff "Extensionsreaktionen" Reaktionsgemische, die wenigstens eine Polymerase, Nukleotide, eine Matrize(n) und Primer umfassen. Die Reaktionsbedingungen sind so gewählt, dass der/die Primer sich an die Matrize anlagern kann/können, und die Polymerase die Verlängerung des/der Primers durch Einbau von matrizenkomplementären Nukleotiden katalysiert. Als Produkt entsteht ein Primerextensionsprodukt.
Eine "Target-Nukleinsäure" ist ein Nukleinsäure-Abschnitt aus einer biologischen Probe, deren Sequenz mit Hilfe des erfindungsgemäßen Verfahrens näher analysiert werden soll. Die biologische Probe besteht dabei in der Regel aus genomischer DNA. Ebensogut ist das erfindungsgemäße Verfahren jedoch für die Analyse von RNA-Sequenzvarianten geeignet. Es ist dabei unerheblich, ob die Probe aus zellulärem Material oder biologischen Flüssigkeiten wie Blut, Serum, Plasma, Urin oder Speichel isoliert wurde.
Unter einer "Sequenzvariante" im Sinne der Erfindung ist eine Target-Nukleinsäure mit einer bestimmten Nukleinsäure-Sequenz zu verstehen, die sich von Sequenzen anderer möglicher Target-Nukleinsäuren nur minimal unterscheidet und bedingt durch diese minimalen Unterschiede identifizierbar ist. Dabei betreffen die Sequenzunterschiede vorzugsweise zwischen ein und drei aufeinander folgende Nukleotidreste. Besonders geeignet ist die vorliegende Erfindung zur Identifikation von Sequenzvarianten betreffend nur einen einzelnen Nukleotidrest (SNP). Dabei kann es sich sowohl um einen Basenaustausch, aber alternativ auch um Nukleotidadditionen bzw. -deletionen handeln. Auf diese Art können verschiedene Allele voneinander unterschieden werden. Punktmutationen oder Polymorphismen können so ebenfalls nachgewiesen werden. Unter einer Sequenzvariante werden somit insbesondere auch Punktmutationen oder Polymorphismen verstanden, die im Hinblick auf prognostische oder diagnostische Fragestellungen analysiert werden.

Bei einer Matrizen-abhängigen Polymerisation von Desoxynukleotid-triphosphaten erfolgt beginnend am 3'-Ende eines sogenannten Primers, welcher an eine einzelsträngige Template-Nukleinsäure hybridisiert ist, eine Polymerisation der Desoxynukleotidtriphosphate in der Weise, dass eine zur Target-Nukleinsäure komplementäre Sequenz entsteht. Derartige Polymerisationsreaktionen in 5'-3'-Orientierung werden vorzugsweise enzymatisch mit sog. DNA-Polymerasen, wie beispielsweise Klenow-Polymerase, durchgeführt. Besonders bevorzugt sind thermostabile DNA-Polymerasen, wie z.B. Taq-Polymerase (Roche Applied Science Katalog Nr. 1146165).
Ein "diskriminierender Primer" im Sinne der Erfindung ist ein Primer, dessen Sequenz exakt komplementär zu einer bestimmten Sequenzvariante ist, wobei diese Sequenz bestimmte Unterschiede zu einer anderen Sequenzvariante aufweist, welche sich in der zu analysierenden Probe befinden kann. Als "diskrimlnierender Nukleotidrest" wird in diesem Zusammenhang ein Nukleotidrest verstanden, dessen Komplement in den verschiedenen existierenden Sequenzvarianten von unterschiedlichen Nukleotidresten gebildet wird.
Der "3'-terminale Nukleotidrest" ist derjenige Nukleotidrest, der sich an dem terminalen Ende eines Oligonukleotidprimers befindet, das eine freie 3'-OH-Gruppe besitzt. Der "proxi-terminale Nukleotidrest" ist derjenige Nukleotidrest eines Oligonukleotidprimers, dessen Ende über eine Phosphatgruppe mit dem 5'-Ende des terminalen Nukleotidrestes verbunden ist. Als proxi-proxi-terminaler Nukleotidrest wird derjenige Nukleotidrest bezeichnet, dessen 3'-Ende über eine Phosphatgruppe mit dem 5'-Ende des proxi-terminalen Nukleotidrests verknüpft ist.

Wie aus der oben stehenden Beschreibung des erfindungsgemäßen Verfahrens hervorgeht, handelt es sich bei den Schritten a)-e) im Wesentlichen um eine Amplifikationsreaktion, die abhängig von der An- oder Abwesenheit einer bestimmten Sequenzreaktion zu einem Amplifikationsprodukt führt. Derartige Verfahren können deshalb nach aus dem Stand der Technik bekannten Protokollen für PCR-Reaktionen durchgeführt werden.
Gegenstand der Erfindung ist in diesem Zusammenhang auch ein Kit, enthaltend Mittel zur Durchführung des erfindungsgemäßen Verfahrens. Ein derartiger Kit enthält insbesondere eine erfindungsgemäße DNA-Polymerase. Wahlweise kann ein derartiger Kit zusätzliche Komponenten enthalten wie beispielsweise einen oder mehrere (diskriminierende) Primer, Desoxynukleotidtriphosphat, Puffer, Quantifizierungsreagenzien, insbesondere interkalierende Reagenzien oder in der kleinen Furche anbindendende Reagenzien, wobei besonders bevorzugt Reagenzien aus der Gruppe von PicoGreen (Molecular Probes), SybrGreen (Molecular Probes), Ethidiumbromid, Gelstar (Cambrex), Vista Green (Amersham) ausgewählt werden, Polymerase-blockierende Antikörper, insbesondere TaqBlock, sowie Mittel zur Template-abhängigen Polymerisation der Desoxynukleotidtriphosphate. Die einzelnen Komponenten des Kits können in verschiedenen Ausführungsformen wahlweise in einem Aufbewahrungsgefäß zusammen bzw. in zwei oder mehreren Aufbewahrungsgefäßen getrennt enthalten sein.

Wie aus den weiter unten aufgeführten Beispielen hervorgeht, sind die beobachteten Effekte hinsichtlich der Verbesserung der Spezifität gegenüber aus dem Stand der Technik verfügbaren Verfahren quantitativ eindeutig belegbar (vgl. Bsp. 7, Fig. 5) und führen dazu, dass unter PCR-Amplifikationsbedingungen Extensionsprodukte von Sequenzvarianten-spezifischen Primern in der Tat im Wesentlichen nur dann erhalten werden, wenn die zu analysierende Probe eine Target-Nukleinsäure mit der nachzuweisenden Sequenzvariante enthält. Dieser spezifische Effekt kann durch Verbesserung und Optimierung der jeweiligen PCR-Parameter mit Hilfe von dem Fachmann aus dem Stand der Technik bekannten Maßnahmen optimiert und an die jeweils nachzuweisende Sequenzvariante adaptiert werden.

Eine weitere Ausführungsform der vorliegenden Erfindung betrifft die Durchführung des erfindungsgemäßen Verfahrens mit Hilfe von Real Time PCR. Bei diese Methodik werden die Endprodukte der Amplifikationsreaktion nicht gelelektrophoretisch nachgewiesen, sondern der Verlauf der Amplifikationsreaktion wird mit Hilfe von geeigneten Fluoreszenz-markierten Hybridisierungssonden verfolgt, so dass kinetische Echtzeitmessungen und Quantifizierungen möglich sind.

Bei den für die erfindungsgemäßen Verfahren einzusetzenden Hybridisationssonden handelt es sich in der Regel um einzelsträngige Nukleinsäuren wie einzelsträngige DNA oder RNA bzw. deren Derivate oder alternativ auch PNAs, die bei der Annealing-Temperatur der Amplifikationsreaktion mit der Target-Nukleinsäure hybridisieren. Üblicherweise haben diese Oligonukleotide eine Länge von 20 bis 100 Nukleotiden.

Die Markierung kann abhängig vom genauen Detektionsformat an jeder beliebigen Ribose- oder Phosphatgruppe des Oligonukleotids eingeführt werden. Bevorzugt sind Markierungen am 5'- und 3'-Ende des Nukleinsäuremoleküls. Die Art der Markierung muß im Echtzeit-Modus der Amplifikationsreaktion detektierbar sein. Dies ist beispielsweise nicht nur mit Fluoreszenzmarkierungen möglich, sondern alternativ auch mithilfe von Markierungen, die nach dem Prinzip der NMR detektierbar sind.

Dabei sind viele verschiedene Testführungen möglich. Als besonders geeignet im Zusammenhang mit der vorliegenden Erfindung haben sich die folgenden drei Detektionsformate erwiesen:
1. FRET-Hvbridisationssonden: Für dieses Testformat werden 2 einzelsträngige Hybridisationssonden gleichzeitig verwendet, die komplementär zu benachbarten Stellen desselben Strangs der amplifizierten Target-Nukleinsäure sind. Beide Sonden sind mit unterschiedlichen Fluoreszenzkomponenten markiert. Bei Anregung mit Licht einer geeigneten Wellenlänge einer ersten Komponente überträgt diese nach dem Prinzip des Fluoreszenzresonanzenergietransfers die absorbierte Energie auf die zweite Komponente, so dass bei Bindung beider Hybridisationsproben an benachbarte Positionen des nachzuweisenden Target-Moleküls eine Fluoreszenzemission der zweiten Komponente gemessen werden kann. Alternativ können ein Fluoreszenz-markierter Primer und nur eine markierte Oligonukleotidsonde verwendet werden (Bernard et al., Analytical Biochemistry 235, 1001-107 (1998)).
2. TaciMan-Hybridisationssonden: Eine einzelsträngige Hybridsidisationssonde wird mit 2 Komponenten markiert. Bei Anregung der ersten Komponente mit Licht einer geeigneten Wellenlänge wird nach dem Prinzip des Fluoreszenz-Resonanzenergietransfers die absorbierte Energie auf die zweite Komponente, den sogenannten Quencher übertragen. Während des Annealing-Schrittes der PCR-Reaktion bindet die Hybridisationssonde an die Target-DNA und wird während der sich anschließenden Elongationsphase durch die 5'-3' Exonuklease-Aktivität der Taq-Polymerase degradiert. Dadurch werden die angeregte Fluoreszenzkomponente und der Quencher räumlich voneinander getrennt, so dass eine Fluoreszenzemission der ersten Komponente gemessen werden kann.
3. Molecular Beacons: Diese Hybridisationssonden sind ebenfalls mit einer ersten Komponente und einem Quencher markiert, wobei sich die Markierungen vorzugsweise an den beiden Enden der Sonde befinden. In Lösung befinden sich beide Komponenten aufgrund der Sekundärstruktur der Sonde in räumlicher Nähe zueinander. Nach Hybridisierung an die Target-Nukleinsäure werden beide Komponenten von einander getrennt, so dass nach Anregung mit Licht einer geeigneten Wellenlänge die Fluoreszenzemission der ersten Komponente gemessen werden kann (Lizardi et al., US 5,118,801).
In alternativen Ausführungsformen kann das jeweilige Amplifkationsprodukt erfindungsgemäß auch durch einen DNA-Bindefarbstoff nachgewiesen werden, welcher bei Interaktion mit doppelsträngiger Nukleinsäure nach Anregung mit Licht einer geeigneten Wellenlänge ein entsprechendes Fluoreszenzsignal emittiert. Als besonders geeignet für diese Anwendung haben sich die Farbstoffe SybrGreen und SybrGold (Molecular Probes) erwiesen. Alternativ können auch interkalierende Farbstoffe verwendet werden.

Die Erfindung wird anhand der nachfolgenden Beispiele und Abbildungen näher erläutert. Die beschriebenen Verfahren sind nicht als Einschränkung der Erfindung sondern als Beispiele zu verstehen, die auch noch nach Modifikationen den Gegenstand der Erfindung beschreiben.

### Beispiele

### Beispiel 1: Aufbau einer Bibliothek und Reinigung von Klenow-Fragment-Varianten

Das Plasmid pQKF (Brakman, S., Nieckchen, P., ChemBioChem 2001, 2, 773-7; siehe auch das in SEQ ID NO:26 gezeigte äquivalente Plasmid pQE30) ermöglicht die Expression des N-terminalen 6-His-markierten Klenow-Fragments von *E.-coli-*DNA-Polymerase I (3'-5'exo⁻) unter der Kontrolle einer T5-Promotor/Doppellac-Operator-Sequenz. Die Einführung von Mutationen in die Motiv-C-Sequenz, die für Q879, V880 und H881 codiert, erfolgte durch eine zweistufige Megaprimer-Mutagenese. PCR-Reaktionen wurden unter Verwendung von PfuTurbo-DNA-Polymerase (Stratagene) und unter Standardbedingungen durchgeführt. Die erste PCR wurde mit einer dotierten Primerbibliothek durchgeführt (5'-GTA CGT ATG ATC ATG NNN NNN NNN GAT GAA CTG GTA TTT-3'; SEQ ID NO:5), die so aufgebaut war, dass sie 40% Nichtwildtyp-Nucleotide auf jeder der neun Zielpositionen und einen 23mer-Downstream-Primer (5'-GCT AAT TAA GCT TGG CTG CAG GC-3'; SEQ ID NO:6) enthielt, was ein 195mer-PCR-Produkt ergab. Die zweite PCR wurde unter Verwendung des Agarose-Gel-gereinigten 195mers und eines 24mer-Antisense-Primers (5'-TAC ATG GAC CTT TAC TTC GAA CGC-3' SEQ ID NO:7) durchgeführt und ergab ein 457-bp-Produkt, das mit Csp45I und HindIII verdaut und in pQKF' einkloniert wurde. Die resultierende Plasmidbibliothek wurde in *E.*-*col*i-XL-1blue (Stratagene) transformiert, Klone wurden aus Agarplatten herausgesucht und getrennt über Nacht in 96-Napf-Platten gezüchtet, die Superbroth-Medium (100 µg/ml Ampicillin) enthielten. Klenow-Fragment-Varianten wurden parallel in 600-µl-Kulturen exprimiert, geerntet und lysiert, wobei man 96-Napf-Platten verwendete, wie es beschrieben ist. Die erhaltenen 300-µl-Lysate wurden mit 900 µl Aufbewahrungspuffer (50mM Tris-HCl pH 7,3, 1 mM DTT, 0,1 mM EDTA, 1 mM PMSF, 1 mM Benzamidin, 1 µg/ml Leupeptin und 1 µg/ml Aprotinin) verdünnt, zentrifugiert und bei -80 °C aufbewahrt.
Für Primerverlängerungsreaktionen und kinetische Messungen im stationären Zustand wurden Klenow-Fragment und ausgesuchte Mutanten exprimiert, wie es oben beschrieben ist, und unter Verwendung von Ni-NTA-Agarose (Qiagen) gereinigt, wobei die Vorschriften des Herstellers befolgt wurden, aber das Imidazol im Lyse- und Waschschritt weggelassen wurde. Die erhaltenen Enzyme waren >95% rein, was durch SDS PAGE mit Coomassie-Blau-Färbung bestätigt wurde. Nach Austausch des Puffers (gegen 100 mM K₂HPO₄, 1 mM DTT pH 6,5 mit 50% Glycerin) wurden Konzentrationen unter Verwendung des Nanoorange-Assays (Molecular Probes) gemessen und auf 1 µg/µl eingestellt.

### Beispiel 2: Durchmusterung (Screening)

Die Reaktionsgemische für die Durchmusterung der Bibliothek enthielten 150 nM Matrize, 225 nM Primer, 50 mM Tris-HCl pH 7,3, 10 mM MgCl₂, 1 mM DTT, 0,05% Triton^{®} X-100 und jeweils 200 µM dNTP. Die Reaktionen umfassten den 20mer-Primer FVL20TH (5'-ACA AAA TAC CTG TAT TCC TT-3'; SEQ ID NO:8), der so gestaltet ist, dass er mit der 3'-terminalen Base an das humane SNP G1691A bindet, das an der Faktor-V-Leiden-Mutation beteiligt ist. Für Messungen von Paarungsverlängerungseffizienzen wurde die 90mer-Matrize TFVL90A (5'-GAC ATC ATG AGA GAC ATC GCC TCT GGG CTA ATA GGA CTA CTT CTA ATC TGT AAG AGC AGA TCC CTG GAC AGG CAA GGA ATA CAG GTA TTT-3'; SEQ ID NO:9), die für das mutante Allel 1691A des humanen Faktor-V-ORF codiert, verwendet, was zu einem TA-Basenpaar am 3'-Terminus des Primers führte. Um Zugang zu Aktivitäten von Klenow-Varianten zu erhalten, die einen fehlgepaarten Primerterminus prozessieren, wurde die 90mer-Matrize TFVL90G (5'-GAC ATC ATG AGA GAC ATC GCC TCT GGG CTA ATA GGA CTA CTT CTA ATC TGT AAG AGC AGA TCC CTG GAC AGG CGA GGA ATA CAG GTA TTT-3' ; SEQ ID NO:10) verwendet, die für das entsprechende Wildtyp-Allel 1691G codiert, was zu einer TG-Fehlpaarung am 3'-Primerterminus führte. Beide Reaktionen wurden für jedes Element der Bibliothek parallel durchgeführt, um eine Bewertung der Aktivitätsverhältnisse als Verlängerungsselektivitäten zu ermöglichen. 10 µl der Reaktionsgemische wurden in schwarze 384-Napf-Platten ausgegeben, die auf 37 °C vorgewärmt wurden, wobei man eine automatische Flüssigkeitshandhabungsvorrichtung (Hamilton Microlab Star) verwendete, und anschließend wurden 5 µl Lysatlösung hinzugefügt. Nach 10 min wurden die Reaktionen durch Zugabe von 30 µl Stopplösung (50 mM Tris-HCl pH 7,3, 100 mM NaCl, 10 mM EDTA) abgebrochen, die 3,4x SYBRGrün I (Molecular Probes) für die Quantifizierung der von Klenow-Varianten erzeugten dsDNA enthielt. Die Fluoreszenzintensitäten wurden mit Hilfe eines Fluoreszenzplatten-Lesegeräts (Polarstar Optima, BMG Labtechnologies GmbH) mit Anregung bei 485 nm und Emission bei 520 nm quantifiziert. Die Verhältnisse der gemessenen Fluoreszenzintensitäten (F_{match}/F_{mismatch}, willkürliche Einheiten) wurden zur Bestimmung der Verlängerungsselektivität herangezogen. Alle DNA-Polymerasen mit größerem Verlängerungsselektivitätsverhältnis (F_{match}/Fₘᵢₛₘₐₜₑₕ) als der Wildtyp wurden als Enzyme identifiziert, die eine erhöhte Verlängerungsselektivität besitzen.

### Beispiel 3: Primerverlängerungsassays

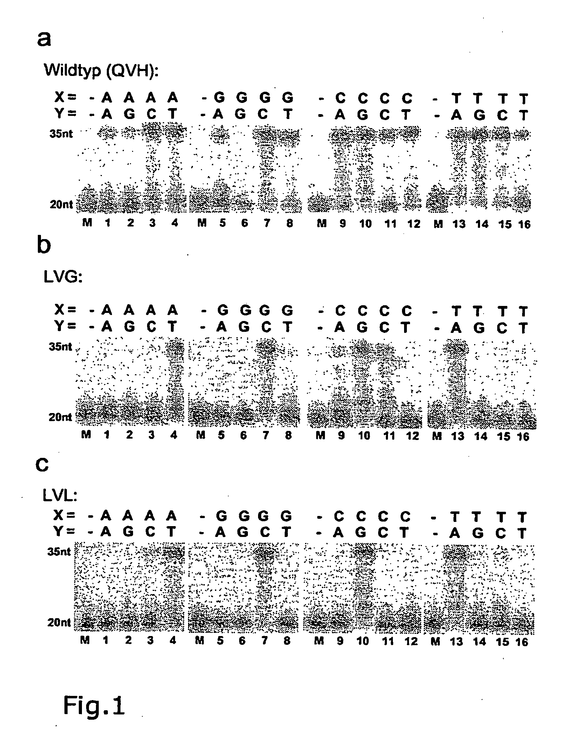

Primer-Matrizensubstrate wurden assoziiert, indem man 5'-³²P-marklerten Primer im spezifischen Reaktionspuffer (50 mM Tris-HCl pH 7,3, 10 mM MgCl₂, 1 mM DTT, 0,05% Triton^{®} X-100) mit der doppelten Menge Matrize mischte. Das Gemisch wurde 5 min lang auf 95 °C erhitzt und anschließend über 1 h auf Raumtemperatur abkühlen gelassen. Nach der Assoziation wurden dNTP hinzugefügt, und die Lösung wurde 5 min lang bei 37 °C inkubiert. 15 µl Reaktionen wurden durch Zugabe von 5 µl Enzymlösung in 1x Reaktionspuffer zu 10 µl Assoziationsmischung eingeleitet, und es wurde 10 min lang bei 37 °C inkubiert. Die Assays beinhalteten 150 nM Primer, 225 nM Matrize, jeweils 1 mM dNTP und 590 nM Enzym in geeignetem Reaktionspuffer. Nach 10 min Inkubation wurden die Reaktionen durch Zugabe von 30 µl Gelbeladungspuffer (80% Formamid, EDTA, 20 mM) abgebrochen, und die Produktgemische wurden durch 14% denaturierendes PAGE analysiert (siehe Fig. 1 und 2). Die folgenden Primer- und Matrizensequenzen wurden im Zusammenhang mit verschiedenen SNPs (Positionen unterstrichen) eingesetzt:
Humane genomische Factor-V-Leiden-DNA-Sequenz: Primer: 5'-ACA AAA TAC CTG TAT TCC TT-3' (SEQ ID NO:11), Wildtypmatrize: 5'-GAT CCC TGG ACA GGC GAG GAA TAC AGG TAT TTT GT-3' (SEQ ID NO:12), mutante Matrize: 5'-GAT CCC TGG ACA GGC AAG GAA TAC AGG TAT TTT GT-3' (SEQ ID NO:12).
Humane somatische BRAF-T1796A-Mutation: Primer: 5'-GAA CCA CTC CAT CGA GAT TTC T-3' (SEQ ID NO:13), Wildtypmatrize: 5'-GGT CTA GCT ACA GTG AAA TCT CGA TGG AGT GGG TC-3' (SEQ ID NO:14), mutante Matrize: 5'-GGT CTA GCT ACA GAG AAA TCT CGA TGG AGT GGG TC-3' (SEQ ID NO:14).
Humane Dihydropyrimidin-Dehydrogenase (DPyD) Mutation G735A: Primer: 5'-GTT TTA GAT GTT AAA TCA CAC TTA T-3' (SEQ ID NO:15), Wildtypmatrize: 5'-CTT TCC AGA CAA CGT AAG TGT GAT TTA ACA TCT AAA AC-3' (SEQ ID NO:16), mutante Matrize: 5'-CTT TCC AGA CAA CAT AAG TGT GAT TTA ACA TCT AAA AC-3' (SEQ ID NO:16).
Humane saure Ceramidase-Mutation A107G: Primer: 5'-CGT TGG TCC TGA AGG AGG AT-3' (SEQ ID NO:17), Wildtypmatrize: 5'-AAA TCA ACC TAT CCT CCT TCA GGA CCA ACG TAC-3' (SEQ ID NO:18), mutante Matrize: 5' -AAA TCA ACC TGT CCT CCT TCA GGA CCA ACG TAC-3' (SEQ ID NO:18).
Getestet wurden so die Mutationen in *E. coli* DNA-Polymerase I (Klenow-Fragment, 5'→3'-Exonuklease-defizient) mit LVL und LVG in Positionen 879-881 (bezogen auf das in SEQ ID NO:2 gezeigte Klenow-Fragment aus *E. coli*) im Vergleich mit dem Wildtyp-Enzym mit QVH in Positionen 879-881 (Fig. 1 und 2).

### Beispiel 4: Klonieren von Tag-DNA-Polymerase

Das Plasmid pTTQ18::*Taq* (SEQ ID NO: 25) wurde von Engelke et al. (Anal. Biochem.1990, 191, 396-400 (1990)) konstruiert und ermöglicht die Expression von *Taq*-DNA-Polymerase unter der Kontrolle einer *Ptac*-Promotor/*lac*-Operator-Sequenz. Die LVL-Mutation wurde durch PCR in das *Taq*-QVH-Motiv eingeführt, wobei man den QuikChange^{®}-Kit von *Stratagene* verwendete. Das resultierende mutante Plasmid und das Wildtypplasmid wurden in *E.*-*coli*-XL1-Blue (*Stratagene*) transformiert. Klone wurden herausgesucht und über Nacht in 20 ml Superbroth (100 µg/ml Carbenicillin) gezüchtet. Die Expression der Taq-Klone wurde in Kulturen in 1 1 Superbroth (100 µg/ml Carbenicillin) durchgeführt, und die Zellen wurden nach 16 h Induktion mit 1 mM IPTG geerntet. Die Reinigung der *Taq*-DNA-Polymerase wurde so durchgeführt, wie es von Engelke et. al (Anal. Biochem.1990, 191, 396-400) beschrieben wird. Anstelle der Reinigung durch Ionenaustausch wurde eine Gelfiltration unter Verwendung einer Säule mit Sephadex^{®}-75 (*Amersham*) angewendet. Die erhaltenen Enzyme waren zu >90% rein, was durch SDS PAGE mit Coomassie-Blau-Färbung bestätigt wurde. Die Konzentrationen wurden unter Verwendung des Nanoorange-Assays (Molecular Probes) und SDS PAGE mit Coomassie-Blau-Färbung gemessen.

### Beispiel 5: Primerverlängerung mit Katalyse durch Taq-DNA-Polymerase

Primer-Matrizensubstrate wurden assoziiert, indem man 5'-³²P-markierten Primer im spezifischen Reaktionspuffer (50 mM Tris -HCl (pH 9,2 bei 25 °C), 16 mM Ammoniumsulfat und 2,5 mM MgCl₂, 0,1 % Tween^{®} 20) mit der doppelten Menge Matrize mischte. Das Gemisch wurde 10 min lang auf 95 °C erhitzt und anschließend über 1 h auf Raumtemperatur abkühlen gelassen. Nach der Assoziation wurden dNTP hinzugefügt, und die Lösung wurde 5 min lang bei 37 °C inkubiert. 15 µl Reaktionen wurden durch Zugabe von 5 µl Enzymlösung in 1x Reaktionspuffer zu 10 µl Assoziationsmischung eingeleitet, und es wurde 10 min lang bei 72 °C inkubiert. Die Assays beinhalteten 150 nM Primer, 225 nM Matrize, jeweils 1 mM dNTP und 0,5 ng *Taq*-LVL-DNA-Polymerase (mutante Polymerase) und 0,06 ng *Taq*-DNA-Polymerase in geeignetem Reaktionspuffer. Nach 10 min Inkubation wurden die Reaktionen durch Zugabe von 30 µl Gelbeladungspuffer (80% Formamid, EDTA, 20 mM) abgebrochen, und die Produktgemische wurden durch 14% denaturierendes PAGE analysiert (siehe Figur 3). Bezüglich der Primer- und Matrizensequenzen, die im Zusammenhang mit den SNPs humane genomische Factor-V-Leiden-DNA-Sequenz, humane somatische BRAF-T1796A-Mutation und humane Dihydropyrimidin-Dehydrogenase (DPyD) Mutation G735A verwendet wurden, wird auf Beispiel 3 verwiesen.

### Beispiel 6: Echtzeit-PCR-Experimente

Echtzeit-PCR wurde unter Verwendung eines *iCycler*-Systems (BIORAD) durchgeführt. Die Reaktionen wurden in einem Gesamtvolumen von 20 µl durchgeführt, das 4 pM der jeweiligen Matrizen in *Taq*-DNA-Polymerase-Puffer (50 mM Tris-HCl (pH 9,2 bei 25 °C), 16 mM Ammoniumsulfat und 2,5 mM MgCl₂, 0,1% Tween^{®} 20 enthielt. Die endgültigen Gemische enthielten dNTPs (jeweils 200 µM dATP, dGTP, dCTP und TTP), Primer (jeweils 0,5 µM der jeweiligen Primersonde und des umgekehrten Primers) und 13 ng *Taq*-DNA-Polymerase (SEQ ID NO:4), 95 ng DNA-Polymerase von *Taq*-LVL-Mutante (SEQ ID NO:4 mit LVL in Positionen 782 - 784) und eine wässrige 1/50.000-Verdünnung einer 10.000fachen Lösung von *SybrGreen I* in DMSO (*Molecular Probes*). Alle PCR-Amplifikationen wurden unter Verwendung des folgenden Programms durchgeführt: Anfangsdenaturierung bei 95 °C während 3 min, dann 40 Zyklen Denaturierung bei 95 °C während 30 s, Primerassoziation bei 55 °C während 35 s und Verlängerung bei 72 °C während 40 s. Die vorgestellten Ergebnisse stammen von wenigstens dreimal wiederholten unabhängigen Messungen an drei Parallelansätzen, die aus einer Stammmischung hervorgingen. Die Ergebnisse sind in Figur 4 zusammengefasst.
Die folgenden DNA-Sequenzen wurden eingesetzt:
Sequenzen im BRAF-Zusammenhang: Primersonde BrafT: 5'-d(GAC CCA CTC CAT CGA GAT TTC T) (SEQ ID NO:19), umgekehrter Primer: 5'-d(AGA GGA AAG ATG AAG TAC TAT G) (SEQ ID NO:20), Zielmatrize BrafX: 5'-d(CAA CTG TTC AAA CTG ATG GGA CCC ACT CCA TCG AGA TTT CXC TGT AGC TAG ACC AAA ATC ACC TAT TTT TAC TGT GAG GTC TTC ATG AAG AAA TAT ATC TGA GGT GTA GTA AGT AAA GGA AAA CAG TAG ATC TCA TTT TCC TAT CAG AGC AAG CAT TAT GAA GAG TTT AGG TAA GAG ATC TAA TTT CTA TAA TTC TGT AAT ATA ATA TTC TTT AAA ACA TAG TAC TTC ATC TTT CCT CT), X= A BrafA, X= T, BrafT (SEQ ID NO:21). Sequenzen im DPyD-Zusammenhang: Primersonde DpyDT: 5'-d(GTT TTA GAT GT TAA ATC ACA CTT AT) (SEQ ID NO:22) , umgekehrter Primer: (5'-d(AAA GCT CCT TTC TGA ATA TTG AG) (SEQ ID NO:23), Zielmatrize DPyDX: 5'-d(AAA ATG TGA GAA GGG ACC TCA TAA AAT ATG TCA TAT GGA AAT GAG CAG ATA ATA AAG ATT ATA GCT TTT CTT TGT CAA AAG GAG ACT CAA TAT CTT TAC TCT TTC ATC AGG ACA TTG TGA CAA ATG TTT CCC CCA GAA TCA TCC GGG GAA CCA CCT CTG GCC CCA TGT ATG GCC CTG GAC AAA GCT CCT TTC TGA ATA TTG AGC TCA TCA GTG AGA AAA CGG CTG CAT ATT GGT GTC AAA GTG TCA CTG AAC TAA AGG CTG ACT TTC CAG ACA ACX TAA GTG TGA TTT AAC ATC TAA AAC), X= A DpyDA, X= T, DpyDG (SEQ ID NO:24). Die Oligonucleotide BrafX und DpyDX (SEQ ID NO:21 und 24) wurden von IBA, Göttingen, synthetisiert und gereinigt.

### Beispiel 7 (Vergleichsbelspiel): Vergleich der Fehlpaarungsdiskriminierung

Verglichen wurden eine erfindungsgemäße und eine literaturbekannte Mutante der *E. coli* DNA-Polymerase I (Klenow-Fragment, 5'→3'-Exonuklease-defizient) in Positionen 879-881 (SEQ ID NO:2) auf die Selektivität der Primerverlängerung. Es handelte sich hierbei um die LVL-Mutante (SEQ ID NO:2 mit LVL in Positionen 879-881) und die QVA-Mutante, welche dem Klenow-Fragment mit H881A aus Minnick, T. et al., J. Biol. Chem. 274, 3067-3075 (1999) entspricht (SEQ ID NO:2 mit HVA in Positionen 879-881).
Mit den beiden genannten Mutanten wurden Primerverlängerungsassays gemäß Beispiel 3 durchgeführt. Getestet wurden die Fehlpaarungsdiskriminierung mit der humanen genomischen Factor-V-Leiden-DNA-Sequenz: Primer: 5'-ACA AAA TAC CTG TAT TCC TT-3' (SEQ ID NO:11), Wildtypmatrize: 5'-GAT CCC TGG ACA GGC GAG GAA TAC AGG TAT TTT GT-3' (SEQ ID NO:12), mutante Matrize: 5'-GAT CCC TGG ACA GGC AAG GAA TAC AGG TAT TTT GT-3' (SEQ ID NO:12). Verglichen wurde die Neigung der beiden Mutanten, Fehlpaarungen im Vergleich zu kanonischen Komplexen zu verlängern. Wie Figur 5 zeigt, hat die QVA-Mutante eine wesentlich höhere Neigung als die LVL-Mutante, Fehlpaarungen zu verlängern.

## Patentansprüche

1. Eine DNA-Polymerase der Familie A, die eine modifizierte Motiv-C-Sequenz und eine, im Vergleich zu der entsprechenden Wildtyppolymerase, erhöhte Fehlpaarungs-Diskriminierung aufweist oder ein Klenow-Fragment derselben, wobei in der Motiv-C-Sequenz QVH in Position 879-881, bezogen auf das in SEQ ID NO:2 gezeigte Klenow-Fragment der *E. coli* DNA-Polymerase, wenigstens der Aminosäurerest Q879 durch einen lipophilen Aminosäurerest ersetzt ist.

2. Die DNA-Polymerase oder deren Klenow-Fragment nach Anspruch 1, die eine bakterielle DNA-Polymerase, vorzugsweise eine thermostabile DNA-Polymerase ist, besonders bevorzugt ausgewählt ist aus der Gruppe von Polymerasen von *Thermus thermophilus, Thermus filiformis, Rhodothermus obamensis* oder *Bacillus stearothermophilus*.

3. Die DNA-Polymerase oder deren Klenow-Fragment nach Anspruch 1 oder 2, wobei in der Motiv-C-Sequenz QVH in Position 879-881
(i) weiterhin H881 durch einen lipophilen Aminosäurerest ersetzt ist, und/oder
(ii) der Aminosäurerest in Position 880 Val, Leu, Ile, Ala oder Tyr, insbesondere Val oder Ile ist.

4. Die DNA-Polymerase oder deren Klenow-Fragment nach einem oder mehreren der Ansprüche 1 bis 3, wobei der lipophile Aminosäurerest ausgewählt ist aus Gly, Ala, Val, Leu, Ile, Pro, Phe, Met und Trp, vorzugsweise ausgewählt ist aus Gly, Ala, Val, Leu und Ile und besonders bevorzugt die Motiv-C-Sequenz QVH ersetzt ist durch die Sequenzen LVL oder LVG.

5. Die DNA-Polymerase oder deren Klenow-Fragment nach einem oder mehreren der Ansprüche 1 bis 4,
(i) die eine Taq-Polymerase mit der in SEQ ID NO:4 gezeigten Sequenz ist, in der die Sequenz QVH in Position 782-784 durch LVL oder LVG ersetzt ist oder
(ii) die ein Klenow-Fragment mit der in SEQ ID NO:2 gezeigten Sequenz ist, in der die Sequenz QVH in Position 879-881 durch LVL oder LVG ersetzt ist.

6. Eine DNA-Sequenz, die für eine DNA-Polymerase oder deren Klenow-Fragment gemäß einem oder mehreren der Ansprüche 1 bis 5 codiert.

7. Ein Vektor, der die DNA-Sequenz gemäß Anspruch 6 enthält.

8. Eine Wirtszelle, die mit dem Vektor gemäß Anspruch 7 transformiert ist und/oder eine DNA gemäß Anspruch 6 aufweist.

9. Ein Verfahren zur Herstellung einer DNA-Polymerase oder deren Klenow-Fragment gemäß einem oder mehreren der Ansprüche 1 bis 5 umfassend das Kultivieren einer Wirtszelle gemäß Anspruch 8 und das Isolieren der DNA-Polymerase oder des Klenow-Fragments aus der Kultur oder dem Kulturüberstand.

10. Verwendung der DNA-Polymerase oder des Klenow-Fragments gemäß einem oder mehreren der Ansprüche 1 bis 5 in diagnostischen und molekularbiologischen *in*-*vitro*-Verfahren, einschließlich allel-spezifischer PCR, DNA-Amplifikation mittels PCR, Kionierung usw.

11. Ein Verfahren zur Bestimmung der An- oder Abwesenheit von mindestens einer Sequenzvariante in einer oder mehreren Target-Nukleinsäuren in einer individuellen Probe, umfassend die Verwendung einer DNA-Polymerase gemäß einem oder mehreren der Ansprüche 1 bis 5.

12. Verfahren gemäß Anspruch 11, enthaltend folgende Schritte:
a) Zugabe von
- Desoxy-Nukleosid-Triphosphaten,
- einer DNA-Polymerase gemäß einem oder mehreren der Ansprüche 1 bis 5,
- mindestens einem diskriminierenden Primer, enthaltend mindestens einen diskriminierenden Nukleotidrest, wobei für jede nachzuweisende Sequenzvariante einer Target-Nukleinsäure ein Primer zugegeben wird, welcher eine Sequenz komplementär zur nachzuweisenden Sequenzvariante aufweist, und wobei die nachzuweisende Sequenzvariante in der Target-Nukleinsäure komplementär zu mindestens einem 3'-terminalen-, 3'-proxi-terminalen- , oder 3'-proxi-proxi-terminalen Nukleotidrest des diskriminierenden Primers ist,
- mindestens einem weiteren Primer, der komplementär ist zu einem Primer-Extensionsprodukt, entstehend durch Extension eines diskriminierenden Primers;
b) Durchführung einer Primer-Extensionsreaktion, wobei ein Verlängerungsprodukt des diskriminierenden Primers im Wesentlichen nur dann erhalten wird, wenn die Probe eine Target-Nukleinsäure mit der nachzuweisenden Sequenzvariante enthält;
c) Trennung der Produkts der Primer-Extensionsreaktion von der Template-Nukleinsäure;
d) Zyklische Wiederholung der Schritte b) und c) zum Erhalt eines Amplifikationsproduktes, beispielsweise durch Polymerase-Kettenreaktion; und
e) Bestimmmung der An- oder Abwesenheit einer Sequenzvariante aufgrund der An- oder Abwesenheit des Amplifikationsproduktes.

13. Das Verfahren nach Anspruch 12, wobei die Schritte b) - e) als Real Time PCR oder Real Time RT-PCR durchgeführt werden.

14. Ein Kit zur Bestimmung der An- oder Abwesenheit von mindestens einer Sequenzvariante in einer oder mehreren Target-Nukleinsäuren in einer individuellen Probe gemäß Anspruch 11 bis 13, enthaltend wenigstens eine DNA-Polymerase gemäß Ansprüchen 1 bis 5.

15. Der Kit nach Anspruch 14, zusätzlich enthaltend einen oder mehrere der folgenden Komponenten:
- einen oder mehrere diskriminierende Primer, enthaltend mindestens einen diskriminierenden Nukleotidrest, wobei die nachzuweisende Sequenzvariante in der Target-Nukleinsäure komplementär zu mindestens einem 3'-terminalen-, 3'-proxi-terminalen-, oder 3'-proxi-proxi-terminalen Nukleotidrest der diskriminierenden Primer ist,
- einen oder mehrere weitere Primer, die komplementär sind zu einem Primer-Extentsionsprodukt, entstehend durch Extension der diskriminierenden Primer,
- Desoxy-Nukleosid-Triphosphate,
- Puffer
- Quantifizierungsreagenzien, insbesondere interkalierende Reagenzien oder in der kleinen Furche anbindende Reagenzien und
- Polymerase-blockierende Antikörper, insbesondere TaqBlock.

## Claims

1. A family A DNA polymerase which has a modified motif C sequence and an enhanced mismatch discrimination as compared to the corresponding wild type polymerase, or a Klenow fragment thereof, wherein in the motif C sequence QVH in positions 879-881, based on the *E. coli* DNA polymerase Klenow fragment shown in SEQ ID NO: 2, at least the amino acid residue Q879 has been replaced by a lipophilic amino acid residue.

2. The DNA polymerase or its Klenow fragment according to claim 1, which is a bacterial DNA polymerase, preferably a thermostable DNA polymerase, more preferably selected from the group consisting of polymerases from *Thermus thermophilus, Thermus filiformis, Rhodothermus obamensis* or *Bacillus stearothermophilus.*

3. The DNA polymerase or its Klenow fragment according to claim 1 or 2, wherein in the motif C sequence QVH in positions 879-881:
(i) H881 has been further replaced by a lipophilic amino acid residue; and/or
(ii) the amino acid residue in position 880 is Val, Leu, Ile, Ala or Tyr, especially Val or Ile.

4. The DNA polymerase or its Klenow fragment according to one or more of claims 1 to 3, wherein said lipophilic amino acid residue is selected from Gly, Ala, Val, Leu, Ile, Pro, Phe, Met and Trp, preferably from Gly, Ala, Val, Leu and Ile, and more preferably the motif C sequence QVH has been replaced by the sequence LVL or LVG.

5. The DNA polymerase or its Klenow fragment according to one or more of claims 1 to 4:
(i) which is a Taq polymerase with the sequence shown in SEQ ID NO: 4 in which the sequence QVH in positions 782-784 has been replaced by LVL or LVG; or
(ii) which is a Klenow fragment with the sequence shown in SEQ ID NO: 2 in which the sequence QVH in positions 879-881 has been replaced by LVL or LVG.

6. A DNA sequence which codes for a DNA polymerase or its Klenow fragment according to one or more of claims 1 to 5.

7. A vector which contains the DNA sequence according to claim 6.

8. A host cell which has been transformed with the vector according to claim 7 and/or includes a DNA according to claim 6.

9. A process for the preparation of a DNA polymerase or its Klenow fragment according to one or more of claims 1 to 5, which comprises culturing a host cell according to claim 8 and isolating the DNA polymerase or the Klenow fragment from the culture or the culture supernatant.

10. Use of the DNA polymerase or the Klenow fragment according to one or more of claims 1 to 5 in diagnostic and molecular-biological *in vitro* methods including allele-specific PCR, DNA amplification by means of PCR, cloning, etc.

11. A method for determining the presence or absence of at least one sequence variant in one or more target nucleic acids in an individual sample, which comprises using a DNA polymerase according to one or more of claims 1 to 5.

12. The method according to claim 11 including the following steps:
a) adding:
- deoxynucleoside triphosphates;
- a DNA polymerase according to one or more of claims 1 to 5;
- at least one discriminating primer containing at least one discriminating nucleotide residue, wherein a primer is added for each sequence variant to be detected of a target nucleic acid, which primer has a sequence complementary to the sequence variant to be detected, and wherein the sequence variant to be detected in the target nucleic acid is complementary to at least one 3'-terminal, 3'-proxi-terminal or 3'-proxi-proxi-terminal nucleotide residue of the discriminating primer;
- at least one other primer which is complementary to a primer extension product formed by extension of a discriminating primer;
b) performing a primer extension reaction wherein an extension product of the discriminating primer is obtained substantially only if the sample contains a target nucleic acid with the sequence variant to be detected;
c) separating the product of the primer extension reaction from the template nucleic acid;
d) reiterating steps b) and c) to obtain an amplification product, for example, by polymerase chain reaction; and
e) determining the presence or absence of a sequence variant from the presence or absence of the amplification product.

13. The method according to claim 12, wherein steps b) to e) are performed as real-time PCR or real-time RT-PCR.

14. A kit for determining the presence or absence of at least one sequence variant in one or more target nucleic acids in an individual sample according to claims 11 to 13, containing at least one DNA polymerase according to claims 1 to 5.

15. The kit according to claim 14, additionally containing one or more of the following components:
- one or more discriminating primers containing at least one discriminating nucleotide residue, wherein the sequence variant to be detected in the target nucleic acid is complementary to at least one 3'-terminal, 3'-proxi-terminal or 3'-proxi-proxi-terminal nucleotide residue of the discriminating primer;
- one or more other primers which are complementary to a primer extension product formed by extension of said discriminating primers;
- deoxynucleoside triphosphates;
- buffers;
- quantification reagents, especially intercalating reagents or reagents binding to the minor groove; and
- polymerase-blocking antibodies, especially TaqBlock.

## Revendications

1. ADN-polymérase de la famille A, qui comprend une séquence de motif C modifiée et une discrimination des appariements augmentée par rapport à la polymérase correspondante de type sauvage, ou fragment de Klenow de cette ADN-polymérase, où, dans la séquence QVH du motif C en position 879-881, par rapport au fragment de Klenow présenté dans SEQ ID N°2 de l'ADN-polymérase de *E. coli*, au moins un résidu d'acide aminé Q879 est remplacé par un résidu d'acide aminé lipophile.

2. ADN-polymérase ou son fragment de Klenow selon la revendication 1, qui est une ADN-polymérase bactérienne, de préférence une ADN-polymérase thermostable choisie d'une manière particulièrement préférée dans le groupe des polymérases de *Thermus thermophilus*, de *Thermus filiformis*, de *Rhodothermus obamensis* ou de *Bacillus stearothermophilus*.

3. ADN-polymérase ou son fragment de Klenow selon la revendication 1 ou 2, où, dans la séquence QVH du motif C en position 879-881,
(i) en outre H881 est remplacé par un résidu d'acide aminé lipophile, et/ou
(ii) le résidu d'acide aminé en position 880 est Val, Leu, Ile, Ala ou Tyr, en particulier Val ou Ile.

4. ADN-polymérase ou son fragment de Klenow selon une ou plusieurs des revendications 1 à 3, où le résidu d'acide aminé lipophile est choisi parmi Gly, Ala, Val, Leu, Ile, Pro, Phe, Met et Trp, de préférence parmi Gly, Ala, Val, Leu et Ile, et d'une manière particulièrement préférée la séquence QVH du motif C est remplacée par les séquences LVL ou LVG.

5. ADN-polymérase ou son fragment de Klenow selon une ou plusieurs des revendications 1 à 4,
(i) qui est une Taq-polymérase ayant la séquence indiquée dans SEQ ID N°4, dans laquelle la séquence QVH en position 782-784 est remplacée par LVL ou LVG, ou
(ii) qui est un fragment de Klenow ayant la séquence indiquée dans SEQ ID N°2, dans laquelle la séquence QVH en position 879-881 est remplacée par LVL ou LVG.

6. Séquence d'ADN qui code pour une ADN-polymérase ou son fragment de Klenow selon une ou plusieurs des revendications 1 à 5.

7. Vecteur qui contient la séquence d'ADN selon la revendication 6.

8. Cellule hôte qui est transformée avec le vecteur selon la revendication 7 et/ou comprend un ADN selon la revendication 6.

9. Procédé pour l'obtention d'une ADN-polymérase ou de son fragment de Klenow selon une ou plusieurs des revendications 1 à 5, qui comprend la culture d'une cellule hôte selon la revendication 8 et l'isolement, à partir de la culture ou du surnageant de culture, de l'ADN polymérase ou du fragment de Klenow.

10. Utilisation de l'ADN-polymérase ou du fragment de Klenow selon une ou plusieurs des revendications 1 à 5 dans des procédés de diagnostic et de biologie moléculaire *in vitro,* y compris la PCR spécifique d'allèle, l'amplification de l'ADN par PCR, le clonage, etc.

11. Procédé pour la détermination de la présence ou de l'absence d'au moins une variante de séquence dans un ou plusieurs acides nucléiques cibles d'un échantillon individuel, comprenant l'utilisation d'une ADN-polymérase selon une ou plusieurs des revendications 1 à 5.

12. Procédé selon la revendication 11, comprenant les étapes suivantes :
a) addition
- de désoxy-nucléoside-triphosphates,
- d'une ADN-polymérase selon une ou plusieurs des revendications 1 à 5,
- d'au moins une amorce discriminante, contenant au moins un résidu nucléotidique discriminant, où, pour chaque variante de séquence à détecter d'un acide nucléique cible, on ajoute une amorce, qui présente une séquence complémentaire de la variante de séquence à détecter, et où la variante de séquence à détecter dans l'acide nucléique cible est complémentaire d'au moins un résidu nucléotidique 3'-terminal, 3'-proxi-terminal ou 3'-proxi-proxi-terminal de l'amorce discriminante,
- d'au moins une amorce supplémentaire qui est complémentaire d'un produit d'extension d'amorce, obtenu par extension d'une amorce discriminante ;
b) exécution d'une réaction d'extension d'amorce, qui permet d'obtenir un produit d'extension de l'amorce discriminante, essentiellement seulement quand la sonde contient un acide nucléique cible ayant la variante de séquence à détecter ;
c) séparation, d'avec l'acide nucléique formant matrice, du produit de la réaction d'extension d'amorce ;
d) répétition cyclique des étapes b) et c) pour obtenir un produit d'amplification, par exemple par une réaction en chaîne de polymérase ; et
e) détermination de la présence ou de l'absence d'une variante de séquence sur la base de la présence ou de l'absence du produit d'amplification.

13. Procédé selon la revendication 12, dans lequel les étapes b) - e) sont mises en oeuvre sous forme d'une PCR en temps réel ou d'une RT-PCR en temps réel.

14. Trousse pour déterminer la présence ou l'absence d'au moins une variante de séquence dans un ou plusieurs acides nucléiques cibles d'un échantillon individuel selon les revendications 11 à 13, contenant au moins une ADN-polymérase selon les revendications 1 à 5.

15. Trousse selon la revendication 14, contenant en outre un ou plusieurs des composants suivants :
- une ou plusieurs amorces discriminantes, contenant au moins un résidu nucléotidique discriminant, où la variante de séquence à détecter se trouvant dans l'acide nucléique cible est complémentaire d'au moins un résidu nucléotidique 3'-terminal, 3'-proxi-terminal ou 3'-proxi-proxi-terminal de l'amorce discriminante,
- une ou plusieurs amorces supplémentaires, qui sont complémentaires d'un produit d'extension d'amorce, obtenu par extension des amorces discriminantes,
- des désoxy-nucléoside-triphosphates,
- un tampon
- des réactifs de quantification, en particulier des réactifs intercalants, ou des réactifs se fixant dans le petit sillon, et
- des anticorps bloquant les polymérases, en particulier TaqBlock.
